# EUROPEAN PATENT APPLICATION

(11) **EP 2 446 848 A1**
(43) Date of publication of application: **02.05.2012**
(21) Application number: 10791936.7
(22) Date of filing: 27.05.2010
(51) Int. Cl.: A61B 18/12, A61B 18/00

(54) **SURGERY INSTRUMENT**

(30) Priority: 26.06.2009 US 492268
(71) Applicant: Olympus Medical Systems Corp., Tokyo 151-0072 (JP)
(72) Inventor: MASUDA, Shinya, Hachioji-shi Tokyo 192-8512 (JP)
(74) Representative: Schicker, Silvia
(86) International application number: PCT/JP2010/059024
(87) International publication number: WO 2010/150618

(57) **Abstract**

A surgical apparatus (1, 181, 191, 201, 211, 221) includes a probe (11) configured to output energy of treating an organism, and a jaw (17) which can be opened/closed with respect to the probe (11). The surgical apparatus (1, 181, 191, 201, 211, 221) includes an output mode switching section (141, 151, 161, 184, 195, 204, 217, 227) configured to selectively switch an output mode between a bipolar mode in which the probe (11) and the jaw (17) are driven as high-frequency electrodes and a probe single output mode in which the energy is output from only the probe (11) in accordance with an open/closed state of the jaw (17).

## Description

### Technical Field

The present invention relates to a surgical apparatus used in treatments such as incision, resection, and coagulation.

### Background Art

Patent Literature 1 discloses a high-frequency treatment apparatus as an example of the surgical apparatus capable of incision, resection, and coagulation of tissues using a high-frequency current.

In the high-frequency treatment apparatus, an operation section provided to a proximal end side is connected to a proximal end portion of an elongated insertion section. An electric cord configured to supply a high-frequency current from a high-frequency cauterization power-supply unit is connected to the operation section. A treatment section configured to treat tissues is disposed in a distal end portion of the insertion section.

A pair of jaws is disposed in the treatment section. An operation rod configured to drive the jaws is inserted into a sheath in a state that the operation rod can advance and retreat in an axial direction. Further, the high-frequency cauterization power-supply unit is electrically connected to the jaws of the treatment section via the operation section and an electric conduction path inside the sheath.

Then, the operation rod is driven in the axial direction in a manner of advancing and retreating in accordance with an operation of the operation section and an opening/closing operation of the jaws is performed in accordance with a movement of the operation rod. At this point, with a closing operation of the jaws, a tissue is gripped between the pair of jaws. With a high-frequency current supplied to the jaws of the treatment section in this state, a high-frequency treatment such as tissue coagulation is carried out.

High-frequency treatment apparatuses are divided into monopolar type treatment apparatuses and bipolar type treatment apparatuses. In a monopolar type treatment apparatus, counter electrode plate is arranged outside the body of a patient when the high-frequency treatment is performed. Then, a high-frequency current is passed to the counter electrode from the treatment apparatus through tissues during the high-frequency treatment. The monopolar treatment apparatus is frequently used when membranous tissues with a low risk of bleeding are quickly treated.

In a bipolar type treatment apparatus, a pair of electrodes, electrically insulated with respect to each other, are provided in the treatment section provided at a distal end of the insertion section. Then, a tissue is heated under a high frequency by passing a high-frequency current between the two electrodes in a state in which the pair of electrodes are simultaneously brought into contact with the tissues. The bipolar treatment apparatus is frequently used when a site likely to bleed is treated or hemostasis of a bleeding site is mainly intended. An example of the bipolar treatment apparatus is shown in Patent Literature 1.

### Citation List

### Patent Literature

Patent Literature 1: Jpn. Pat. Appln. KOKAI Publication No. 2005-237574

### Summary of Invention

### Technical Problem

When a high-frequency treatment apparatus is used in treatment, an appropriate high-frequency treatment apparatus is selected in accordance with the site to be treated, the type of treatment, the situation of use and the like. Then, treatment using a monopolar treatment apparatus and treatment using a bipolar treatment apparatus are used for different purposes based on necessity. However, there are cases when a situation in which it is preferable to use a bipolar treatment apparatus during treatment using a monopolar treatment apparatus arises or conversely, a situation in which it is preferable to use a monopolar treatment apparatus during treatment using a bipolar treatment apparatus arises. In such a case, a treatment has to be continued by interchanging a monopolar treatment apparatus and a bipolar treatment apparatus. Thus, it becomes necessary to spend time in replacing the treatment apparatuses, which may make the treatment time of the whole surgery longer. Accordingly, operability of operation by the user may be deteriorated.

The present invention has been developed in view of the above circumstances and an object thereof is to provide a surgical apparatus capable of providing appropriate high-frequency treatment in accordance with the situation of use and the like and reducing the treatment time of the whole surgery by improving operability of operation by the user.

### Solution to Problem

To solve above mentioned problems, according to one aspect of the invention, a surgical apparatus includes a probe configured to output energy of treating an organism; a jaw which can be opened/closed with respect to the probe; and an output mode switching section configured to selectively switch an output mode between a bipolar mode in which the probe and the jaw are driven as high-frequency electrodes and a probe single output mode in which the energy is output from only the probe in accordance with an open/closed state of the jaw.

According to one another aspect of the invention, a surgical apparatus includes a probe configured to output energy of treating an organism; a jaw which can be opened/closed with respect to the probe; an operation section configured to operate the jaw to open/close; and an output mode switching section configured to selectively switch an output mode to one of a probe single output mode in which the energy is output only from the probe and a bipolar mode in which each of the probe and the jaw is driven as a high-frequency electrode, wherein the output mode switching section includes: a movable member configured to move in accordance with an operation of the operation section; and a contact switching section configured to perform an opening/closing operation of a contact, in which a conduction state of a high-frequency current to the jaw is connected or cut off, in accordance with a movement of the movable member.

According to one another aspect of the invention, a surgical apparatus includes a probe configured to output energy of treating an organism; a jaw which can be opened/closed with respect to the probe; and an output mode switching mechanism configured to switch an output mode between a bipolar mode in which the probe and the jaw are used as electrodes and a probe single output mode in which the energy is output from only the probe, wherein the output mode switching mechanism includes an output mode switching mechanism configured to switch to the bipolar mode when the jaw performs a closing operation and configured to switch to the probe single output mode when the jaw performs an opening operation in accordance with an opening/closing operation of the jaw.

### Advantageous Effects of Invention

According to the present invention, a surgical apparatus capable of providing appropriate high-frequency treatment in accordance with the situation of use and the like and reducing the treatment time of the whole surgery by improving operability of operation by the user can be provided.

### Brief description of Drawings

FIG. 1 is a perspective view showing an outline configuration of a whole ultrasonic treatment device according to a first embodiment of the present invention;
FIG. 2 is a perspective view showing a state in which connecting portions of the ultrasonic treatment device according to the first embodiment are removed;
FIG. 3A is a plan view showing a distal end part of a sheath unit of the ultrasonic treatment device according to the first embodiment;
FIG. 3B is a plan view showing a distal end part of a probe unit of the ultrasonic treatment device according to the first embodiment;
FIG. 4A is a longitudinal sectional view showing the distal end part of the sheath unit of the ultrasonic treatment device according to the first embodiment;
FIG. 4B is a longitudinal sectional view showing an insulation coating of an inner circumferential surface of an internal cylinder of the sheath unit according to the first embodiment;
FIG. 5 is a V-V line sectional view of FIG. 4A;
FIG. 6 is a VI-VI line sectional view of FIG. 4A;
FIG. 7 is a VII-VII line sectional view of FIG. 4A;
FIG. 8 is a longitudinal sectional view showing a proximal end part of the sheath unit of the ultrasonic treatment device according to the first embodiment;
FIG. 9A is an IXA-IXA line sectional view of FIG. 8;
FIG. 9B is an IXB-IXB line sectional view of FIG. 8;
FIG. 10 is an X-X line sectional view of FIG. 8;
FIG. 11 is an XI-XI line sectional view of FIG. 8;
FIG. 12 is a perspective view showing a connecting capsule of the sheath unit of the ultrasonic treatment device according to the first embodiment;
FIG. 13 is a side view showing the connecting capsule of the sheath unit of the ultrasonic treatment device according to the first embodiment;
FIG. 14 is a side view showing a connected state of a handle unit and a vibrator unit of the ultrasonic treatment device according to the first embodiment;
FIG. 15 is a longitudinal sectional view showing a unit connecting part of the ultrasonic treatment device according to the first embodiment;
FIG. 16 is a longitudinal sectional view showing an internal configuration of the handle unit of the ultrasonic treatment device according to the first embodiment;
FIG. 17A is a 17-17 line sectional view of FIG. 16 showing a state before the handle unit and the sheath unit of the ultrasonic treatment device according to the first embodiment are engaged;
FIG. 17B is a 17-17 line sectional view of FIG. 16 showing a state after the handle unit and the sheath unit of the ultrasonic treatment device according to the first embodiment are engaged;
FIG. 18 is an 18-18 line sectional view of FIG. 16;
FIG. 19 is a 19-19 line sectional view of FIG. 16;
FIG. 20 is a 20-20 line sectional view of FIG. 16;
FIG. 21 is a 21-21 line sectional view of FIG. 16;
FIG. 22 is a 22-22 line sectional view of FIG. 16;
FIG. 23 is a 23-23 line sectional view of FIG. 16;
FIG. 24 is a 24-24 line sectional view of FIG. 16;
FIG. 25 is a 25-25 line sectional view of FIG. 16;
FIG. 26 is a perspective view showing an electrode holding member of the ultrasonic treatment device according to the first embodiment;
FIG. 27 is a front view showing the electrode holding member of the ultrasonic treatment device according to the first embodiment;
FIG. 28 is a side view showing the electrode holding member of the ultrasonic treatment device according to the first embodiment;
FIG. 29 is a perspective view showing an electrode member of the ultrasonic treatment device according to the first embodiment;
FIG. 30 is a cross sectional view showing the electrode member of the ultrasonic treatment device according to the first embodiment;
FIG. 31 is a perspective view showing the state before rotating engagement in which the handle unit and the sheath unit of the ultrasonic treatment device according to the first embodiment are connected;
FIG. 32 is a plan view showing the state before rotating engagement in which the handle unit and the sheath unit of the ultrasonic treatment device according to the first embodiment are connected;
FIG. 33 is a perspective view showing the state after rotating engagement in which the handle unit and the sheath unit of the ultrasonic treatment device according to the first embodiment are connected;
FIG. 34 is a plan view showing the state after rotating engagement in which the handle unit and the sheath unit of the ultrasonic treatment device according to the first embodiment are connected;
FIG. 35 is a side view showing a state before an assembling member is assembled to a base member of a fixed handle of the handle unit of the ultrasonic treatment device according to the first embodiment;
FIG. 36 is a plan view showing the probe unit of the ultrasonic treatment device according to the first embodiment;
FIG. 37 is a 37-37 line sectional view of FIG. 36;
FIG. 38 is a plan view showing a connected state of the vibrator unit and a cable of the ultrasonic treatment device according to the first embodiment;
FIG. 39 is a plan view showing a proximal end portion of the vibrator unit and the cable of the ultrasonic treatment device according to the first embodiment;
FIG. 40 is a schematic diagram showing electric paths of the vibrator unit of the ultrasonic treatment device according to the first embodiment;
FIG. 41 is a longitudinal sectional view showing the internal configuration of a distal end portion of the vibrator unit of the ultrasonic treatment device according to the first embodiment;
FIG. 42 is a longitudinal sectional view of principal parts showing a state in which an output mode switching section of the ultrasonic treatment device according to the first embodiment is switched to a probe single output mode;
FIG. 43 is a longitudinal sectional view of principal parts showing a state of being switched to a bipolar mode of the ultrasonic treatment device according to the first embodiment;
FIG. 44 is a block diagram schematically showing an electric circuit of a whole system of the ultrasonic treatment device according to the first embodiment;
FIG. 45 is a side view of principal parts showing a state in which a handpiece of the ultrasonic treatment device according to the first embodiment is used as a monopolar treatment apparatus;
FIG. 46 is a longitudinal sectional view showing an internal configuration of a handpiece of an ultrasonic treatment device according to a second embodiment of the present invention;
FIG. 47 is a longitudinal sectional view of principal parts showing a state in which an output mode switching section of the handpiece of the ultrasonic treatment device according to the second embodiment is switched to a probe single output mode;
FIG. 48 is a longitudinal sectional view of principal parts showing a state in which the output mode switching section of the handpiece of the ultrasonic treatment device according to the second embodiment is switched to a bipolar mode;
FIG. 49 is a longitudinal sectional view showing an internal configuration of a handpiece of an ultrasonic treatment device according to a third embodiment of the present invention;
FIG. 50 is a longitudinal sectional view of principal parts showing a state in which an output mode switching section of the handpiece of the ultrasonic treatment device according to the third embodiment is switched to a probe single output mode;
FIG. 51 is a longitudinal sectional view of principal parts showing a state in which the output mode switching section of the handpiece of the ultrasonic treatment device according to the third embodiment is switched to a bipolar mode;
FIG. 52 is a block diagram schematically showing an electric circuit of a whole system of a surgical apparatus according to a fourth embodiment of the present invention;
FIG. 53 is a block diagram schematically showing an electric circuit of a whole system of a surgical apparatus according to a fifth embodiment of the present invention;
FIG. 54 is a block diagram schematically showing an electric circuit of a whole system of a surgical apparatus according to a sixth embodiment of the present invention;
FIG. 55 is a block diagram schematically showing an electric circuit of a whole system of a surgical apparatus according to a seventh embodiment of the present invention; and
FIG. 56 is a block diagram schematically showing an electric circuit of a whole system of a surgical apparatus according to an eighth embodiment of the present invention.

### Description of Embodiments

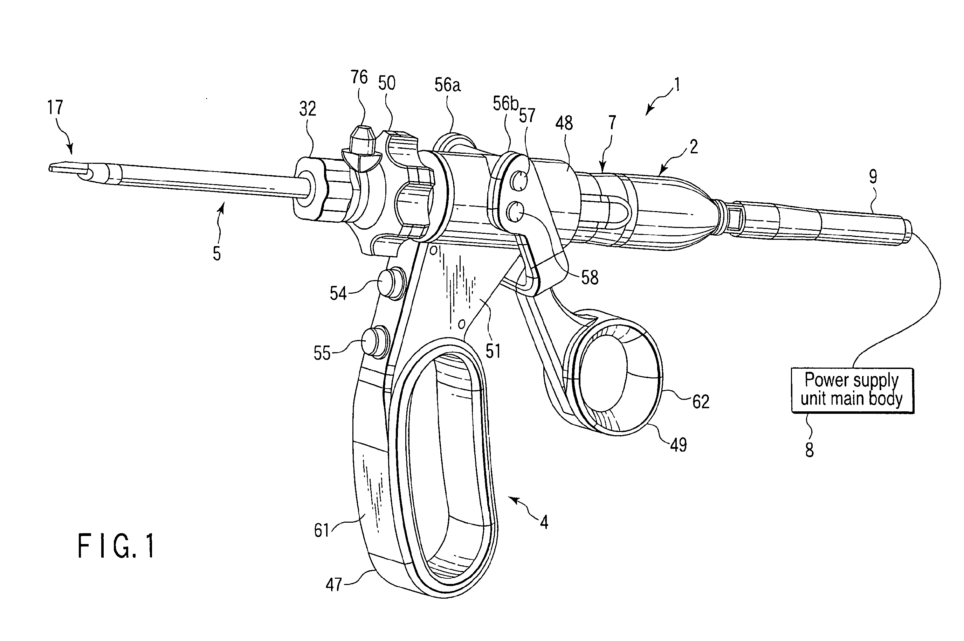

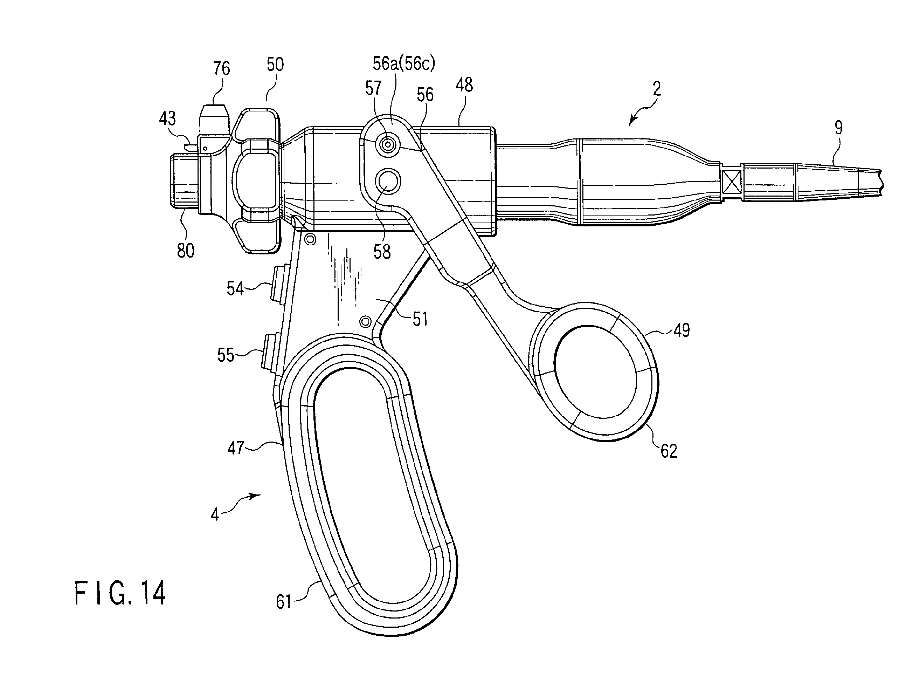

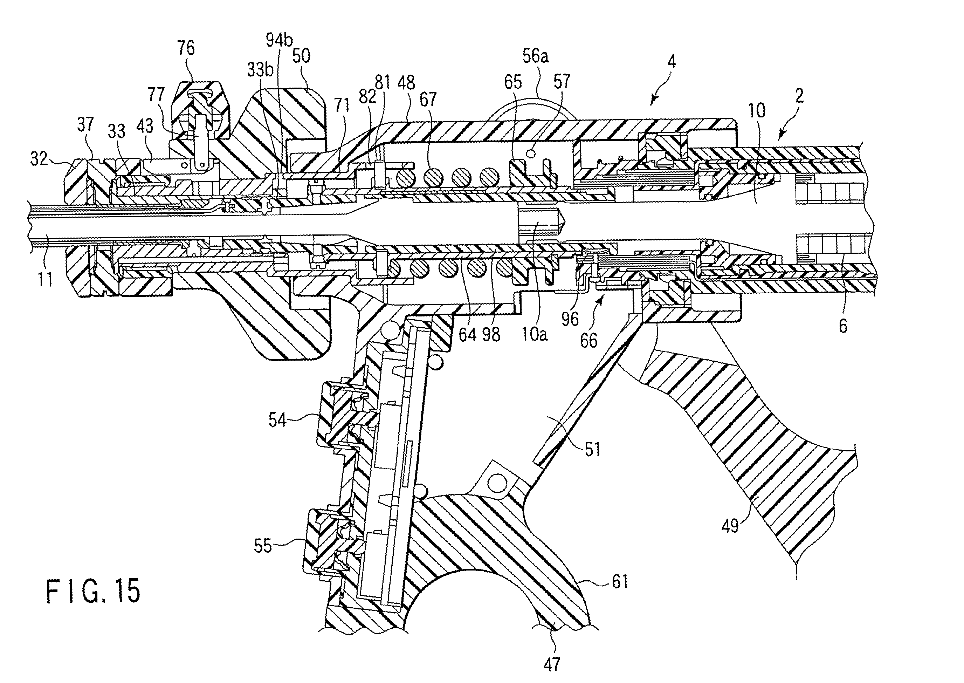

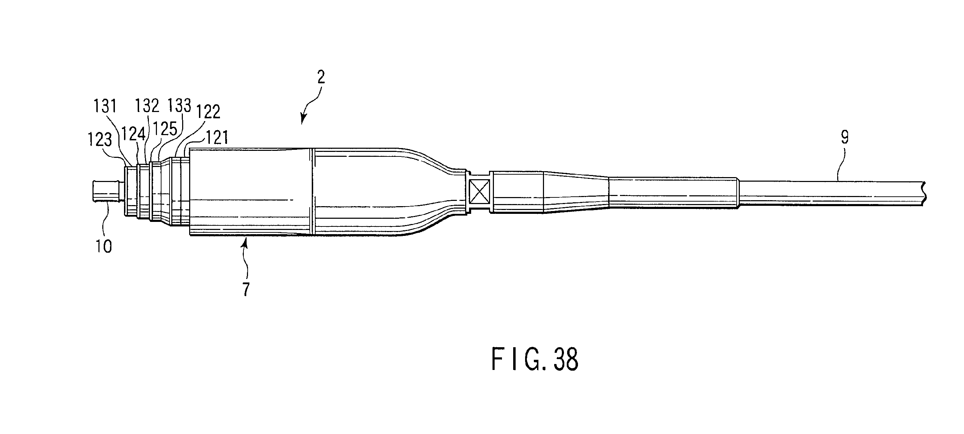

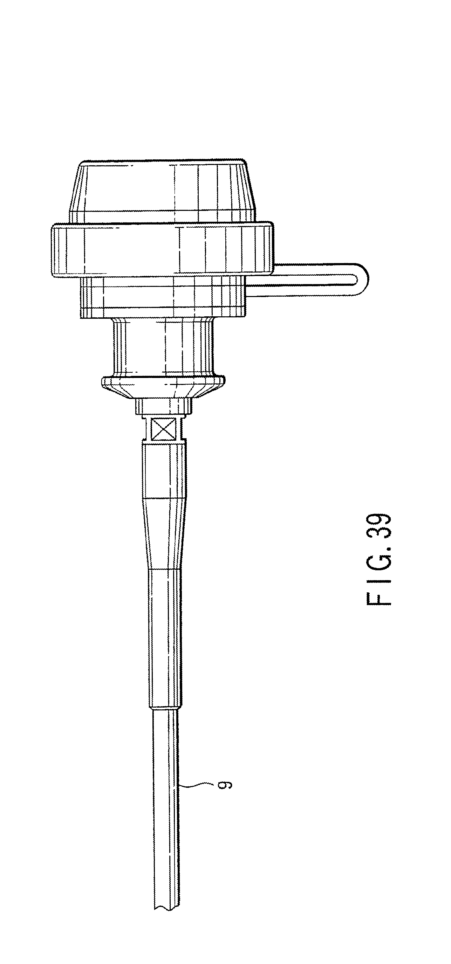

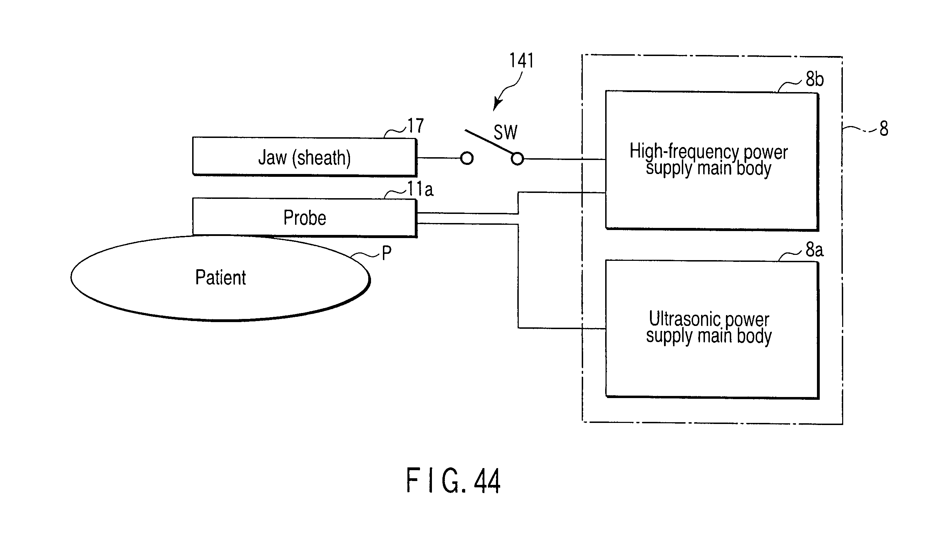

The first embodiment of the present invention will be described below with reference to FIGS. 1 to 45. FIG. 1 is a diagram showing an outline configuration of a whole handpiece 1 of an ultrasonic treatment device, which is a surgical apparatus of the present embodiment. The ultrasonic treatment device according to the present embodiment is an ultrasonic coagulation/incision treatment device capable of treatment such as incision, resection, and coagulation of tissues by using ultrasonic waves and also capable of treatment using a high-frequency current. FIG. 44 is a block diagram schematically showing an electric circuit of a whole system of the ultrasonic treatment device.

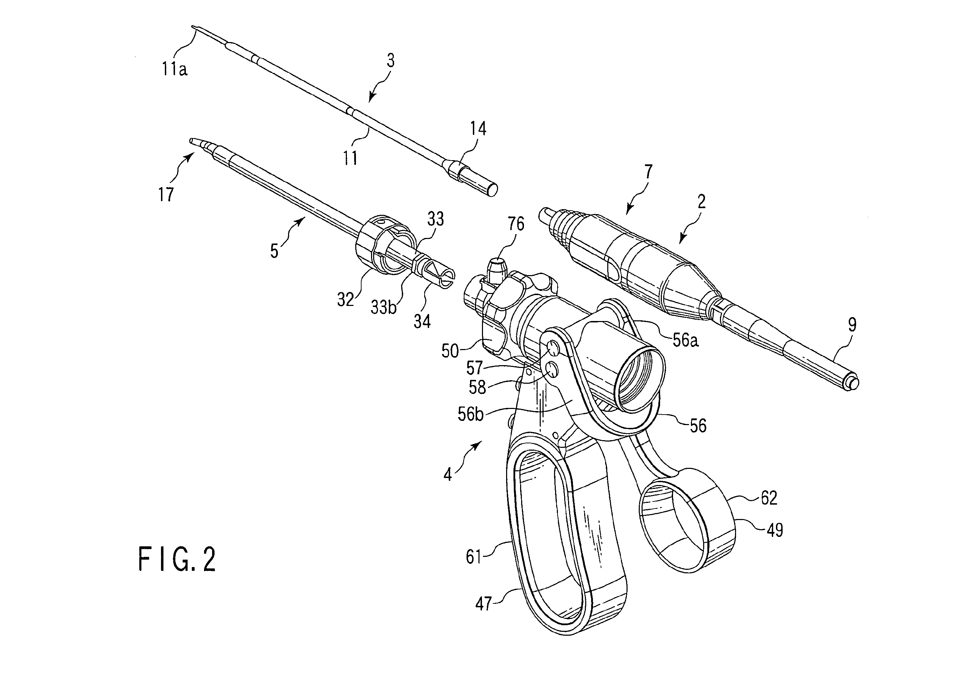

As shown in FIG. 2, the handpiece 1 includes four units that are a vibrator unit (ultrasonic output section) 2, a probe unit 3, a handle unit 4, and a sheath unit 5. These units are connected in such that each unit can be removed.

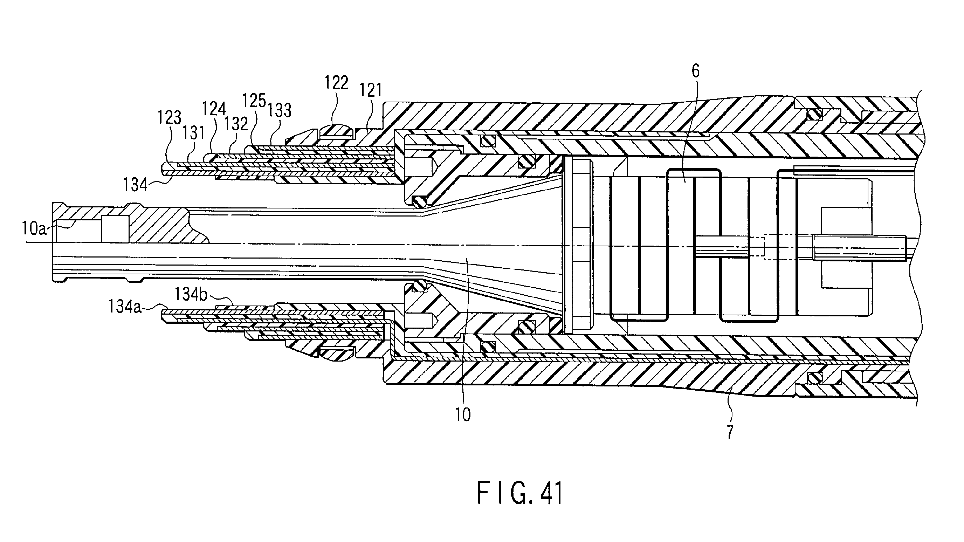

Inside the vibrator unit 2, a vibrator 6 (see FIG. 41), described later, which is configured to generate an ultrasonic vibration by piezoelectric elements configured to convert a current into an ultrasonic vibration is provided. An outer side of the piezoelectric elements is covered with a cylindrical vibrator cover 7. Further, one end of a cable 9 is connected to a proximal end of the vibrator unit 2.

The other end of the cable 9 is connected to a power supply unit main body 8. As shown in FIG. 44, the power supply unit main body 8 includes an ultrasonic power supply main body 8a configured to supply a current to generate an ultrasonic vibration and a high-frequency power supply main body 8b configured to supply the high-frequency current.

A proximal end portion of a horn 10 configured to increase amplitude of an ultrasonic vibration is connected to a distal end portion of the ultrasonic vibrator 6 inside the vibrator cover 7. A screw hole portion 10a of mounting a probe is formed in a distal end portion of the horn 10.

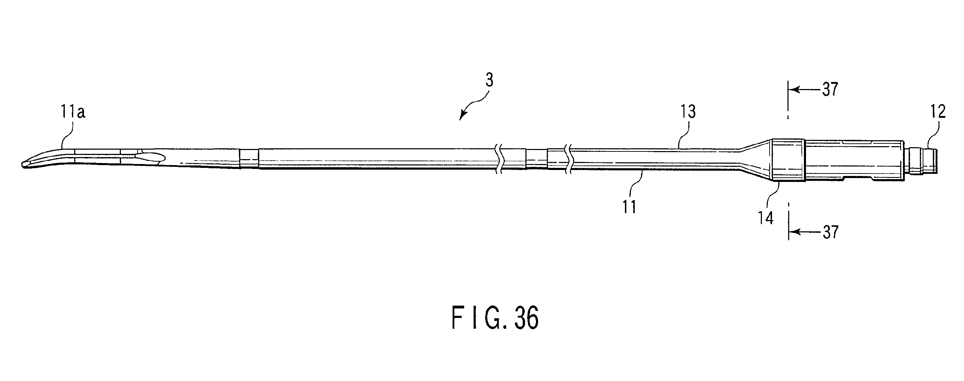

FIG. 36 is a diagram showing an appearance of the whole probe unit 3. The probe unit 3 is designed to have a whole length that is an integral multiple of half the wavelength of the ultrasonic vibration. The probe unit 3 includes a rod probe (vibration transmission member) 11 made of metal. A screw portion 12 screwed into the screw hole portion 10a of the horn 10 is provided in a proximal end portion of the probe 11. The screw portion 12 is screwed into the screw hole portion 10a of the horn 10 of the vibrator unit 2. Accordingly, assembly between the probe unit 3 and the vibrator unit 2 is completed. A first high-frequency electric path 13 on which the high-frequency current can be transmitted is formed in a connected body of the ultrasonic vibrator 6 and the probe unit 3.

A probe distal end 11a is provided in a distal end portion of the probe 11. The probe distal end 11a is formed as a curved shape of a substantial J shape. The vibration necessary in treatment by the probe distal end 11a is obtained by reducing a cross section in the axial direction of the probe unit 3 at several locations of node positions of vibration along the axial direction thereof. A rubber ring formed of an elastic member in a ring shape is mounted in several locations of the node positions of vibration along the axial direction of the probe unit 3. By providing the rubber ring, interference between the probe unit 3 and the sheath unit 5 is prevented.

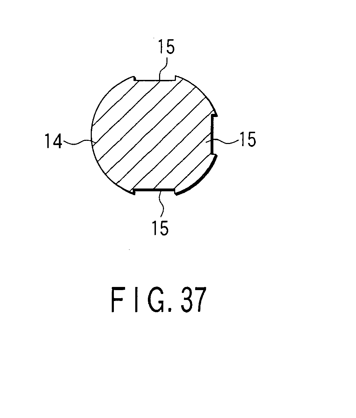

A flange portion 14 is provided in a most proximal end side node position of vibration closest in the axial direction of the probe unit 3. As shown in FIG. 37, an engaging recess 15 in a key groove shape is formed in three locations in a circumferential direction on an outer circumferential surface of the flange portion 14.

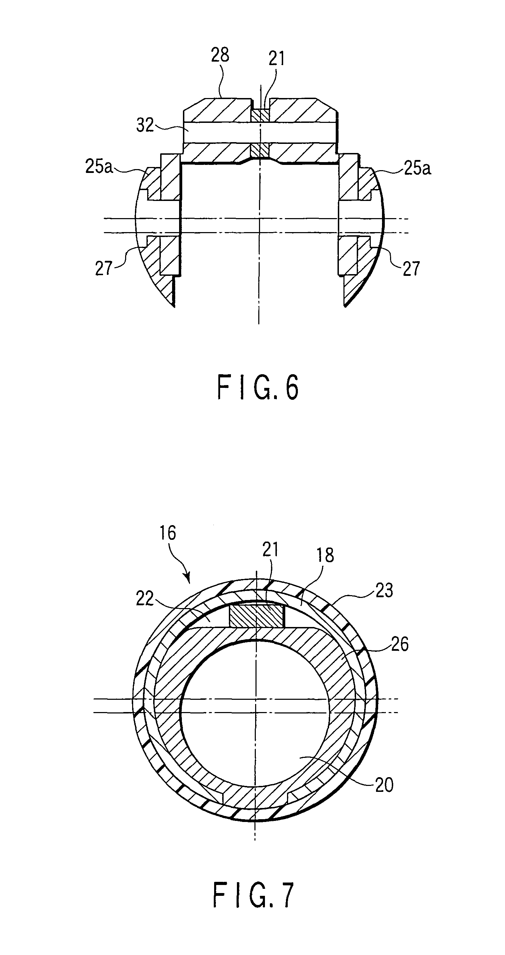

The sheath unit 5 includes a sheath main body 16 formed in a cylindrical shape and a jaw 17 disposed at a distal end of the sheath main body 16. As shown in FIG. 7, the sheath main body 16 includes an external cylinder 18 whose sectional shape is circular and which is made of metal, and an internal cylinder 19 whose sectional shape is non-circular such as a D shape and which is made of metal. A channel 22 through which a drive shaft 21 of the jaw 17 is inserted is formed between the external cylinder 18 and the internal cylinder 19.

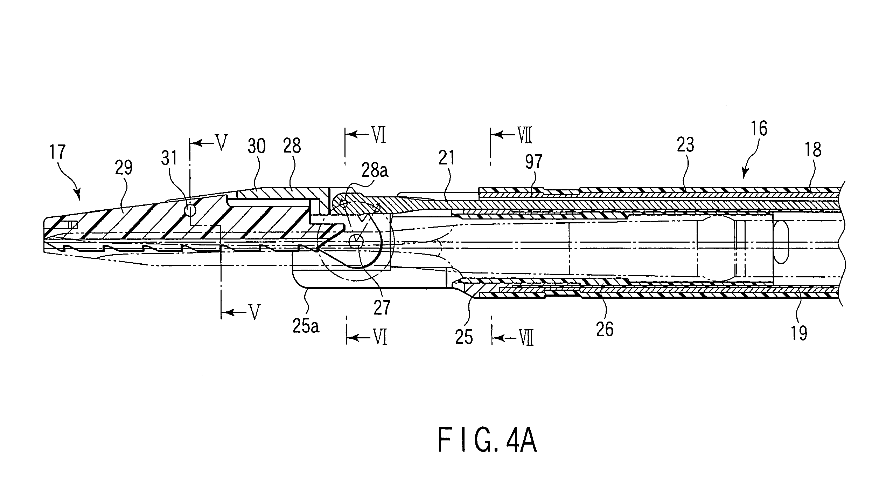

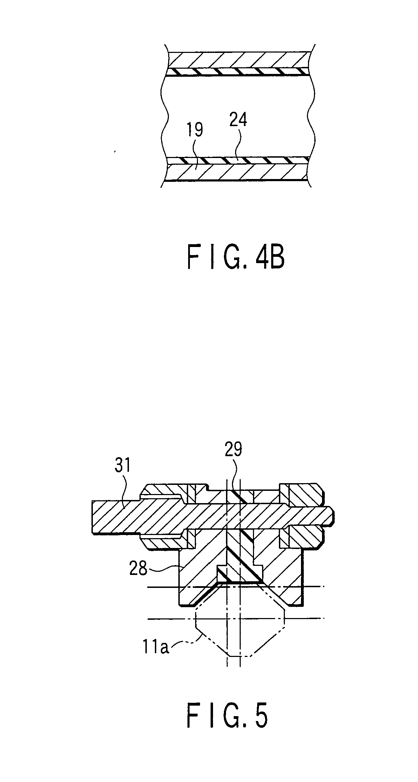

As shown in FIG. 4A, an outer circumferential surface side of the external cylinder 18 is covered with an insulation tube 23. As shown in FIG. 4B, an insulation coating 24 is formed of an insulation material on an inner circumferential surface of the internal cylinder 19. Instead of the insulation coating 24, an insulation tube may be provided to an inner circumferential surface side of the internal cylinder 19. With the insulation coating 24 of the internal cylinder 19, the probe unit 3 and the sheath unit 5 are electrically insulated.

A proximal end portion of a tip cover 25 in a substantially cylindrical shape is fixed to a distal end portion of the external cylinder 18. A pressing member 26 in a pipe shape configured to press the probe unit 3 in such that the probe unit 3 does not come into contact with the tip cover 25 is mounted to the inner circumferential surface side of the proximal end portion of the tip cover 25. A channel 20 through which the probe unit 3 is inserted and whose sectional shape is circular is formed to the inner side of the pressing member 26.

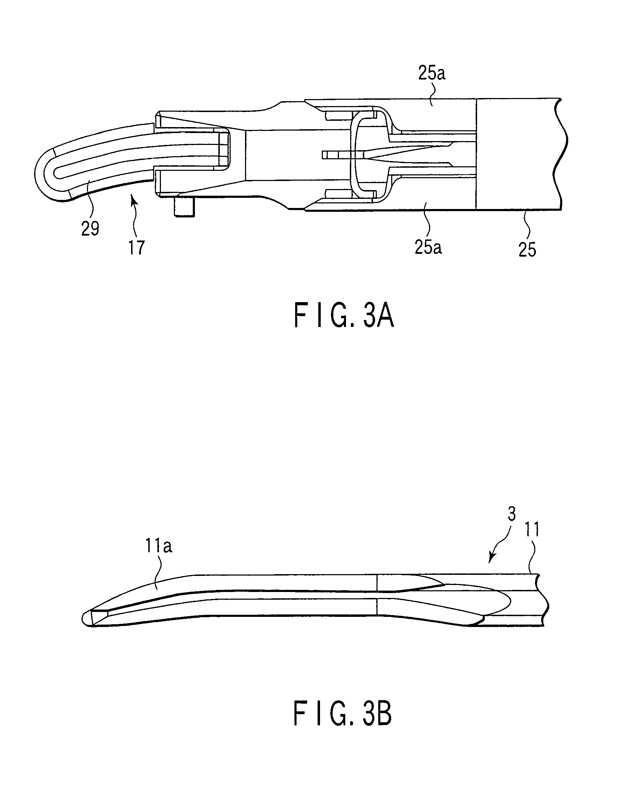

As shown in FIG. 3A, a pair of left-and-right jaw support portions 25a are provided to extend toward the distal side in a distal end portion of the tip cover 25. As shown in FIG. 6, metallic jaw main body 28 of the jaw 17 is rotatably mounted on the jaw support portions 25a via two fulcrum pins 27. As shown in FIG. 3A, the jaw 17 is formed in a curved shape of a substantial J shape corresponding to the probe distal end 11a of the probe unit 3. The jaw 17 is face to the probe distal end 11a of the probe unit 3 and is rotatably supported around the two fulcrum pins 27 (see FIG. 6). The jaw 17 is configured to be operated to perform an opening action in which the jaw 17 is rotated in a direction away from the probe distal end 11a of the probe unit 3 or a closing action in which the jaw 17 is rotated in a direction toward the probe distal end 11a of the probe unit 3. With the closing action of the jaw 17, tissues are gripped between the jaw 17 and the probe distal end 11a of the probe unit 3.

The jaw main body 28 includes a gripping member 29 formed of a resin material such as PTFE and a metallic holding member 30 configured to hold the gripping member 29. The gripping member 29 is mounted on the holding member 30 by a pin 31 in such that the holding member 30 is rotatable by a specified angle (see FIG. 5). Further, as shown in FIG. 4A, a distal end portion of the drive shaft 21 is connected to a proximal end of the jaw main body 28 via a pin 28a. The drive shaft 21 passes through the tip cover 25 and, as shown in FIG. 7, passes between the external cylinder 18 and the internal cylinder 19 of the sheath main body 16 to extend up to the proximal side of the sheath main body 16.

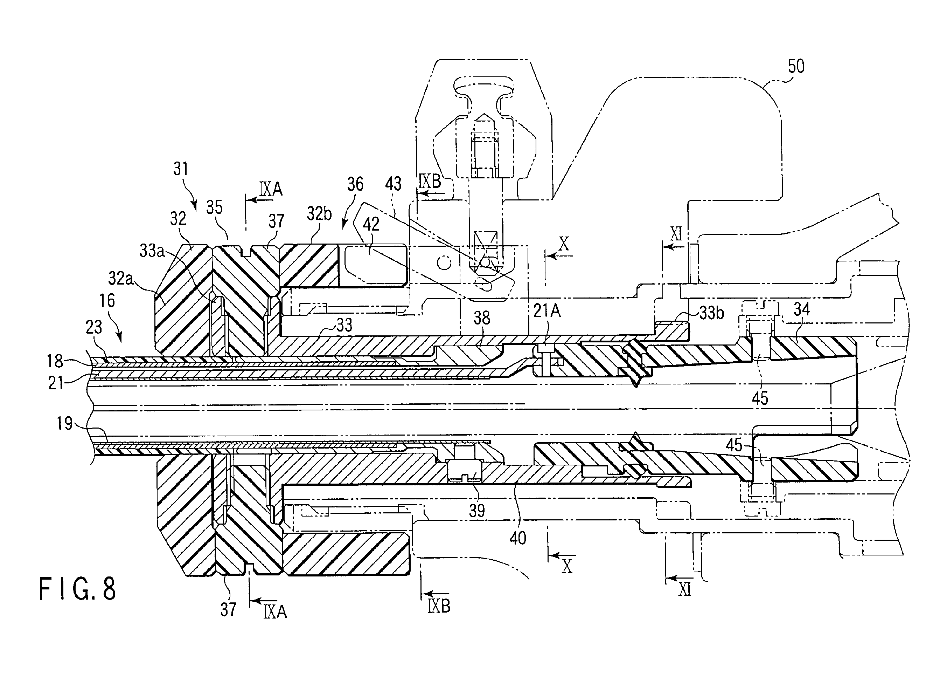

FIG. 8 is a diagram showing the proximal end portion of the sheath main body 16. A removal mechanism 31 detachably attached to the handle unit 4 is provided in the proximal end portion of the sheath main body 16. The removal mechanism 31 includes a knob member 32 in a cylindrical shape formed of a resin material and having a relatively large radius, a guide cylinder 33 which is a cylindrical member made of metal, and a connecting capsule 34 in a cylindrical shape formed of a resin material.

The knob member 32 includes a first fixed portion 32a in a ring shape provided in a distal end portion thereof and a second fixed portion 32b in a cylindrical shape provided in a proximal end portion thereof. The first fixed portion 32a is fixed to the outer circumferential surface of the proximal end portion of the sheath main body 16. The second fixed portion 32b of the knob member 32 includes a fixed portion 35 of the guide cylinder 33 provided in a distal side site and a removal section 36 of the handle unit 4 provided in a proximal side site.

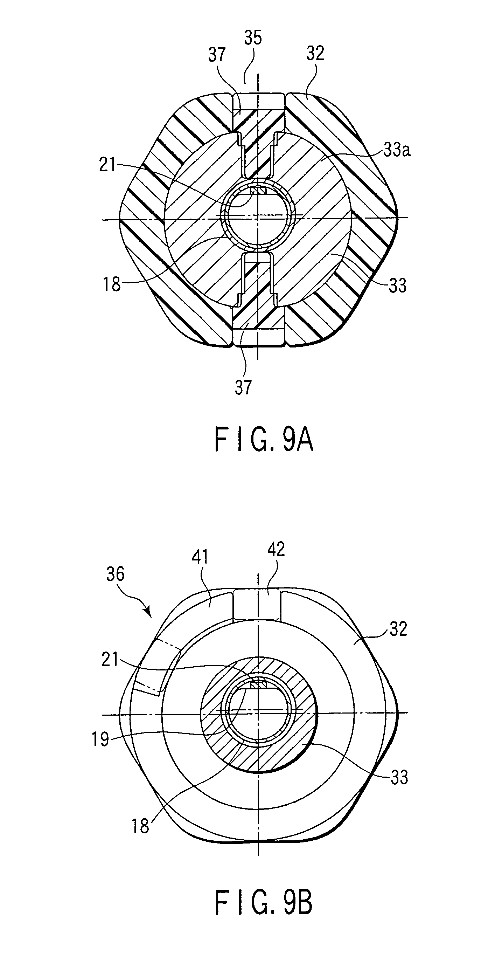

The guide cylinder 33 includes a tip flange portion 33a provided in a distal end portion thereof and having a relatively large radius, and an outer circumferential flange portion 33b provided in a proximal side site thereof. As shown in FIG. 9A, the tip flange portion 33a of the guide cylinder 33 is fixed to the knob member 32 by two fixing screws 37 made of resin in a state that the tip flange portion 33a is inserted into the knob member 32.

A connecting tube 38 made of metal is disposed to the inner side of the guide cylinder 33. An inner circumferential surface of a distal end portion of the connecting tube 38 is fixed to the external cylinder 18 of the sheath main body 16 by laser welding. Further, the connecting tube 38 and the guide cylinder 33 are fixed by a fixing screw 39 made of metal. Accordingly, the guide cylinder 33, the fixing screw 39, the connecting tube 38, the external cylinder 18, the tip cover 25, the fulcrum pin 27, and the jaw main body 28 are electrically conducted to form a sheath-unit-side electric path 40 on which a high-frequency current is transmitted.

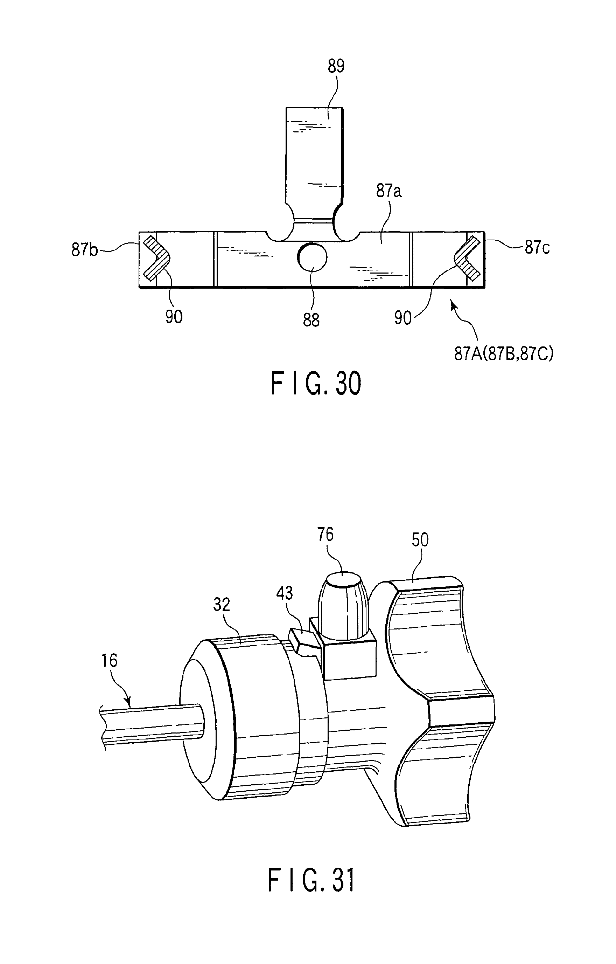

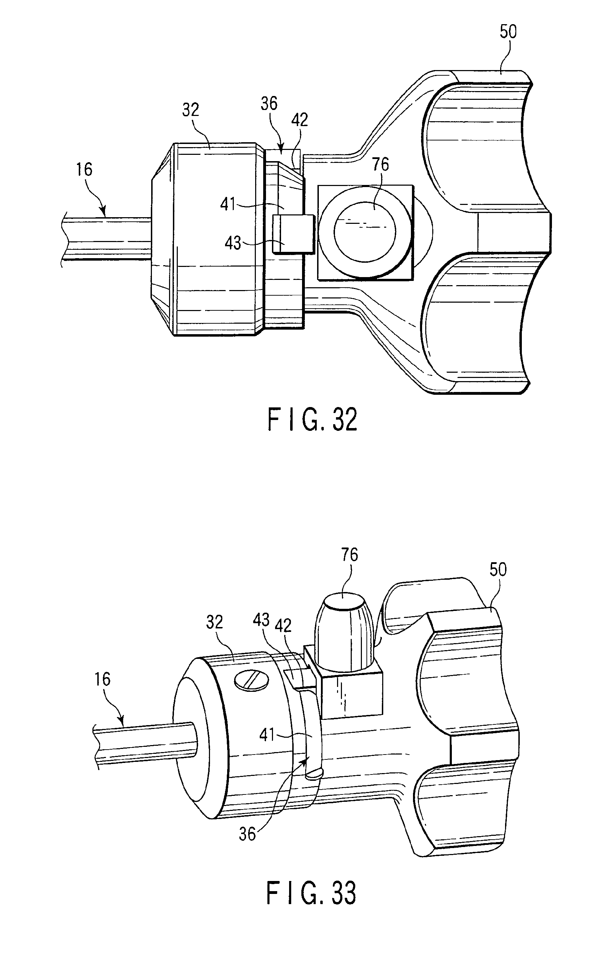

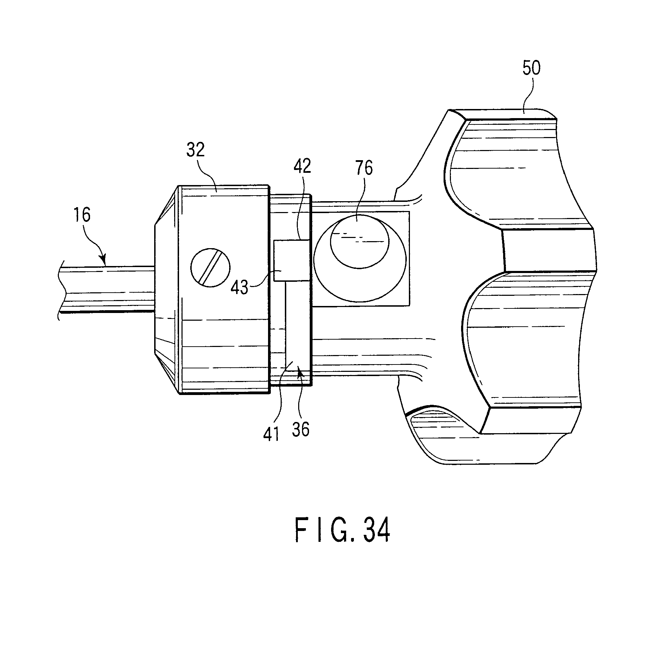

As shown in FIG. 9B, the removal section 36 of the knob member 32 includes a guide groove 41 like an inclined plane provided to be extended along the circumferential direction, and an engaging recess 42 formed at one end of the guide groove 41. The guide groove 41 includes a tapering inclined plane whose outside diameter becomes smaller toward the proximal side of the knob member 32. The engaging recess 42 is formed of a recess having a radius smaller than that of the inclined plane of the guide groove 41. An engaging lever 43, described later, provided on the side of the handle unit 4 is engaged with the engaging recess 42 in a manner allowing disengagement. FIGS. 33 and 34 are diagrams showing a state in which the engaging lever 43 is engaged with the engaging recess 42, and FIGS. 31 and 32 are diagrams showing a disengaged state in which the engaging lever 43 is extracted from the engaging recess 42.

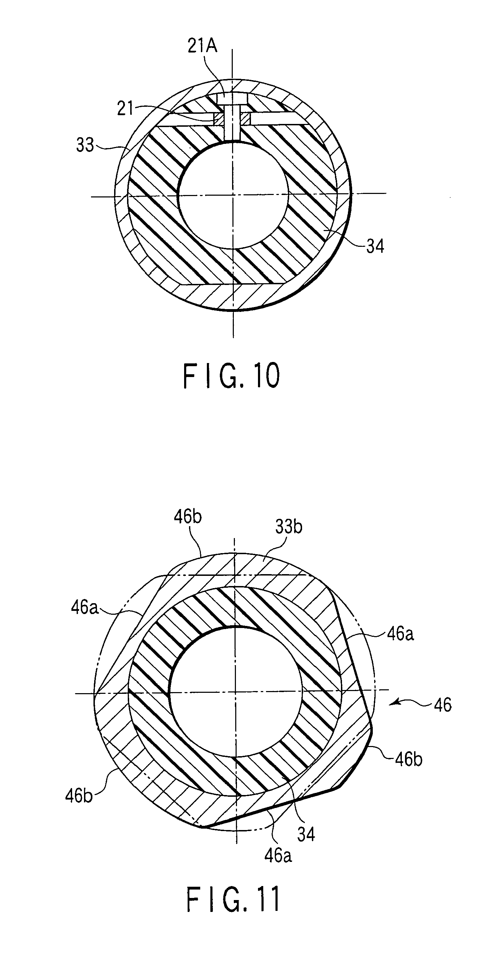

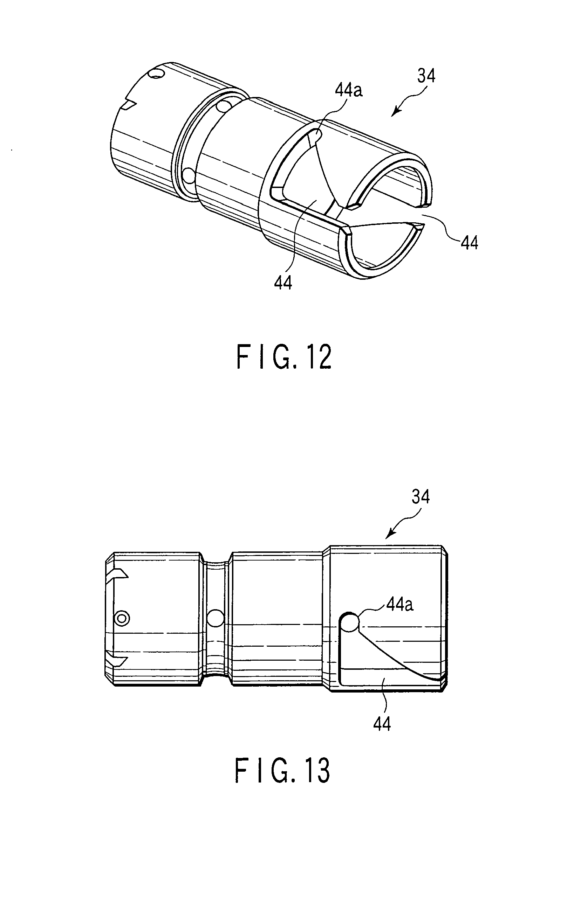

The connecting capsule 34 is inserted into the guide cylinder 33 slidably in the axial direction of the sheath main body 16. The proximal end portion of the drive shaft 21 is fixed to a distal end portion of the connecting capsule 34 via a pin 21A (see FIG. 10). As shown in FIGS. 12 and 13, two guide grooves 44 are provided in a proximal end portion of the connecting capsule 34. An engaging pin 45, described later, provided on the side of the handle unit 4 is engaged with the guide groove 44 in a manner allowing disengagement. An engaging groove 44a configured to restrict the movement of the engaging pin 45 in the axis direction of the sheath main body 16 is formed in a termination portion (proximal end portion) of the guide groove 44.

As shown in FIG. 11, the outer circumferential flange portion 33b includes a non-circular engaging portion 46. In the engaging portion 46, three flat portions 46a which is formed by notching an outer circumferential surface of the outer circumferential flange portion 33b, whose section is circular, at a plurality of locations (three locations in the present embodiment) in the circumferential direction are provided. A corner portion 46b having a diameter larger than that of the flat portion 46a is formed in each of joint portions between the three flat portions 46a. Accordingly, the engaging portion 46 whose sectional shape is substantially triangular is formed in the outer circumferential flange portion 33b. The engaging portion 46 in a non-circular shape need not necessarily be substantially triangular and may be, for example, polygonal such as quadrangular or pentagonal and various non-circular shapes can be considered.

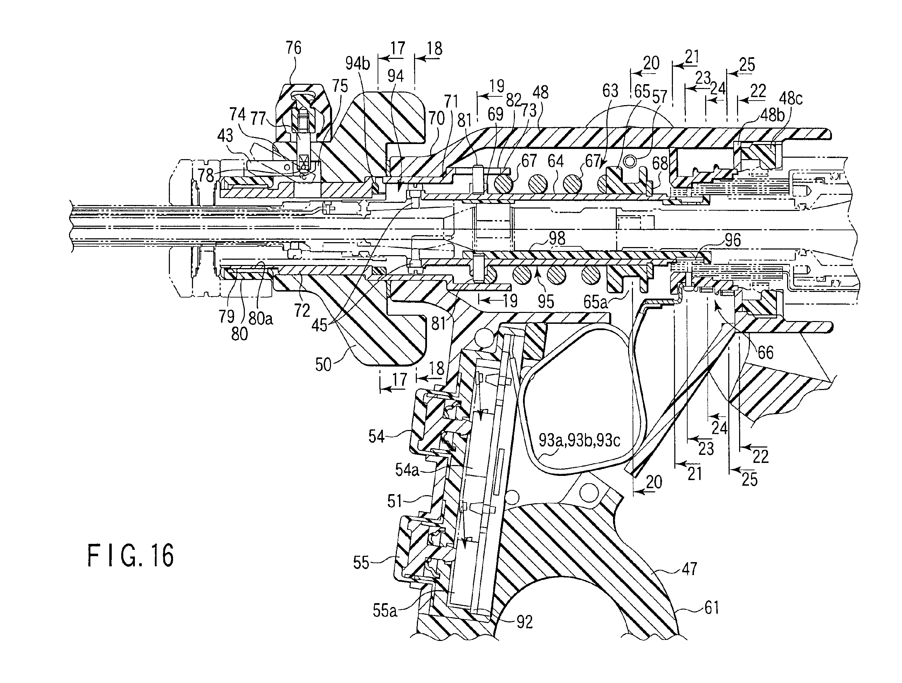

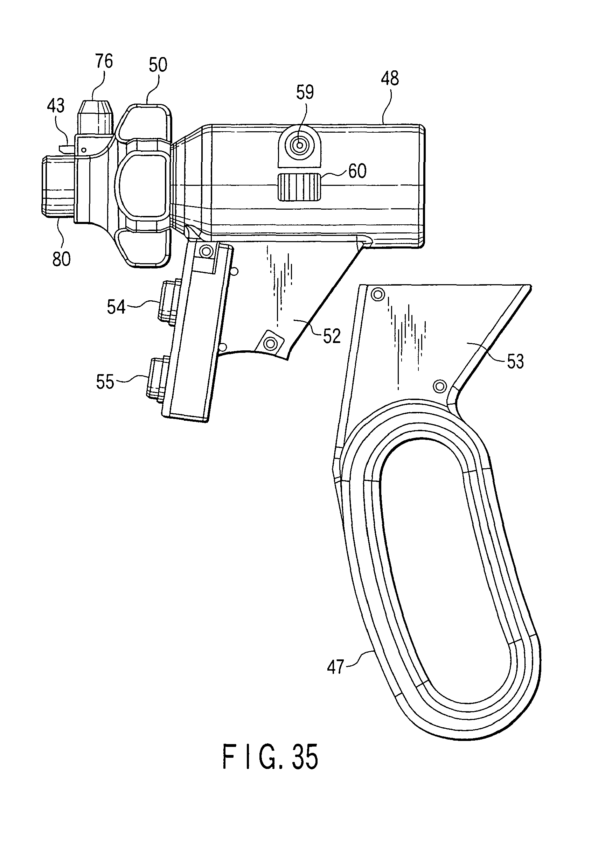

The handle unit 4 includes a fixed handle 47, a holding cylinder 48, a movable handle 49, a rotation operation knob 50, and a handle unit-side electric path 95 on which a high-frequency current is transmitted. The holding cylinder 48 is disposed to an upper side of the fixed handle 47. A switch holding section 51 is provided between the fixed handle 47 and the holding cylinder 48. As shown in FIG. 35, the switch holding section 51 includes a switch mounting portion 52 fixed to a lower end portion of the holding cylinder 48 and a cover member 53 fixed to an upper end portion of the fixed handle 47. The switch mounting portion 52 includes a plurality (two in the present embodiment) of hand switch buttons (for example, an incision switch button 54 and a coagulation switch button 55), which are push button switches. As shown in FIG. 16, an incision switch 54a configured to be operated by the incision switch button 54, a coagulation switch 55a configured to be operated by the coagulation switch button 55, and a wiring circuit board 92 are incorporated into the switch mounting portion 52. An incision wire 93a whose one end is connected to the incision switch 54a, a coagulation wire 93b whose one end is connected to the coagulation switch 55a, and a ground wire 93c whose one end is connected to a ground common terminal are connected to the wiring circuit board 92. The three wires 93a to 93c are incorporated into the switch holding section 51 in a rolled form.

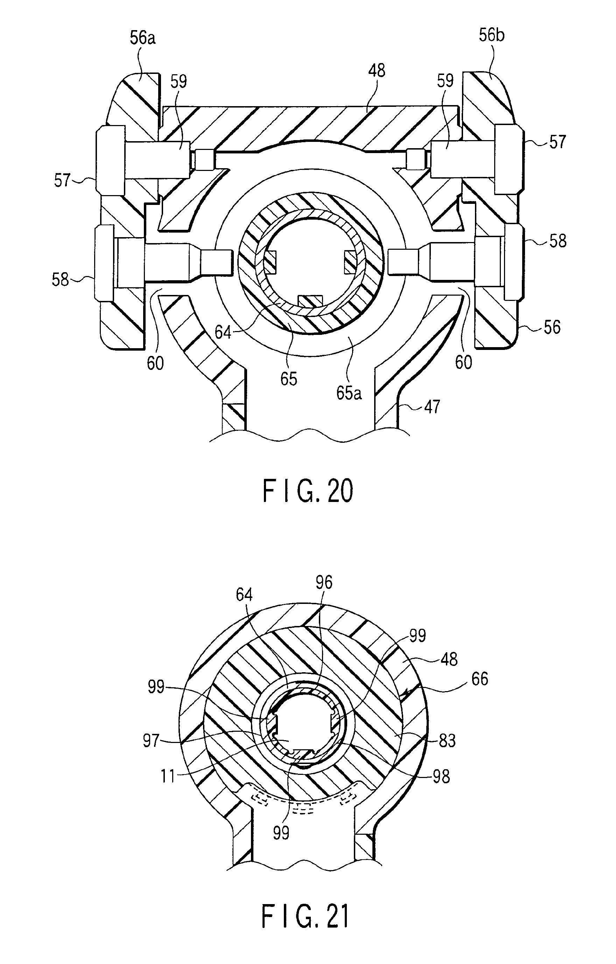

The movable handle 49 includes an arm portion 56 in an approximate U shape provided in an upper part thereof. As shown in FIG. 20, the U-shaped arm portion 56 includes two arms 56a, 56b. The movable handle 49 is assembled into the holding cylinder 48 in a state that the holding cylinder 48 is inserted between the arms 56a, 56b.

A fulcrum pin 57 and an action pin 58 are mounted on each of the arm portions 56a, 56b. A pin receiving hole portion 59 and a window portion 60 are formed in each of both side portions of the holding cylinder 48. The fulcrum pin 57 of each of the arm portions 56a, 56b is inserted into the corresponding pin receiving hole portion 59 of the holding cylinder 48. Accordingly, an upper end portion of the movable handle 49 is rotatably and pivotally supported by the holding cylinder 48 via the fulcrum pin 57.

A finger insertion portion 61 is provided on a lower end portion of the fixed handle 47 and a finger insertion portion 62 is provided on a lower end portion of the movable handle 49. By rotating the movable handle 49 around the fulcrum pin 57 in a state that fingers are inserted to the finger touching portions 61, 62, an opening/closing operation of the movable handle 49 is performed with respect to the fixed handle 47.

Each of the action pins 58 of the movable handle 49 passes through the corresponding window portion 60 of the holding cylinder 48 to be extended into the holding cylinder 48. An operation force transmission mechanism 63 configured to transmit an operation force of the movable handle 49 to the drive shaft 21 of the jaw 17 is provided inside the holding cylinder 48.

As shown in FIG. 16, the operation force transmission mechanism 63 includes a metallic spring receiving member 64 in a cylindrical shape and a resin slider member 65. The spring receiving member 64 is arranged coaxially with a center line of the holding cylinder 48 and provided to be extended in an insertion direction of the probe unit 3.

A proximal end portion of the spring receiving member 64 is connected to a contact unit 66 in a cylindrical shape, described later, which is fixed to the proximal end portion of the holding cylinder 48 in a state that the spring receiving member is rotatable in a direction around the axis and can advance and retreat in the insertion direction of the probe unit 3. A pair of engaging pins 45, described above, provided on the side of the handle unit 4 are provided in a distal end portion of the spring receiving member 64 to project toward an inner direction. When the handle unit 4 and the sheath unit 5 are connected, each of the pair of engaging pins 45 provided on the side of the handle unit 4 is engaged with the corresponding engaging groove 44a provided in the termination portion (proximal end portion) of the guide groove 44 of the sheath unit 5 in a manner allowing to disengage.

A coil spring 67, the slider member 65, a stopper 68, and a spring receiver 69 are provided on the outer circumferential surface of the spring receiving member 64. A distal end portion of the coil spring 67 is fixed to the spring receiver 69. The stopper 68 is configured to control the movement position of the slider member 65 toward the proximal side. The coil spring 67 is mounted between the spring receiver 69 and the slider member 65 with a specified mounting capacity.

An engaging groove 65a in a ring shape is formed on an outer circumferential surface of the slider member 65 along the circumferential direction. As shown in FIG. 20, the action pins 58 of the movable handle 49 are engaged with the engaging groove 65a by being inserted thereinto. When the movable handle 49 is gripped to operate to close the movable handle 49 with respect to the fixed handle 47, the action pins 58 rotate around the fulcrum pin 57 in accordance with a rotating operation of the movable handle 49. The slider member 65 moves forward in the distal direction along the axial direction in accordance with the rotating movement of the action pins 58. In this case, the spring receiving member 64 linked to the slider member 65 via the coil spring 67 also advances or retreats together with the slider member 65. Accordingly, an operation force of the movable handle 49 is transmitted to the connecting capsule 34 via the pair of engaging pins 45 and the drive shaft 21 of the jaw 17 moves forward in the distal direction. Then, the jaw main body 20 of the jaw 17 rotates around the fulcrum pins 27.

Further, when tissues are gripped between the gripping member 29 of the jaw 17 and the probe distal end 11a of the probe unit 3 by a closing operation of the movable handle 49 with respect to the fixed handle 47, the gripping member 29 rotates by a specified angle around the pin 31 as a fulcrum by following a deformation of the probe distal end 11a. Accordingly, a uniform force is applied over the entire length of the gripping member 29. By outputting ultrasonic waves in this state, tissues such as blood vessels can be coagulated or dissected (cut).

A bearing 70 in a ring shape is formed in the distal end portion of the holding cylinder 48. A metallic rotation transmission member 71 in a cylindrical shape is connected to the bearing 70 rotatably in the direction around the axis. The rotation transmission member 71 includes a projection portion 72 projecting to the distal side of the bearing 70 and a large-diameter portion 73 extended from the bearing 70 to the inner side of the holding cylinder 48.

The rotation operation knob 50 is fixed to the projection portion 72 by fitting from outside. The engaging lever 43 is disposed in a distal end portion of the rotation operation knob 50. A central portion of the engaging lever 43 is rotatably connected to the projection portion 72 via a pin 74. A root portion of the engaging lever 43 is extended to the inner side of a lever accommodation recess 75 formed on a distal surface of the rotation operation knob 50.

An operation button 76 configured to operate the engaging lever 43 in a disengagement direction is disposed on an outer circumferential surface of the tip portion of the rotation operation knob 50. An action pin 77 projecting downward is provided on the operation button 76. The action pin 77 passes through wall hole of the rotation operation knob 50 to be extended into the inner side of the lever accommodation recess 75. The root portion of the engaging lever 43 is rotatably connected to a lower end portion of the action pin 77 via a pin 78.

A safety ring 80 of the rotation operation knob 50 is disposed in a tip portion of the projection portion 72. A male screw portion 79 is formed in the tip portion of the projection portion 72. A female screw portion 80a screwed into the male screw portion 79 is formed on the inner circumferential surface of the safety ring 80. The rotation operation knob 50 is fixed to the rotation transmission member 71 by the female screw portion 80a of the safety ring 80 being screwed into the male screw portion 79 of the projection portion 72.

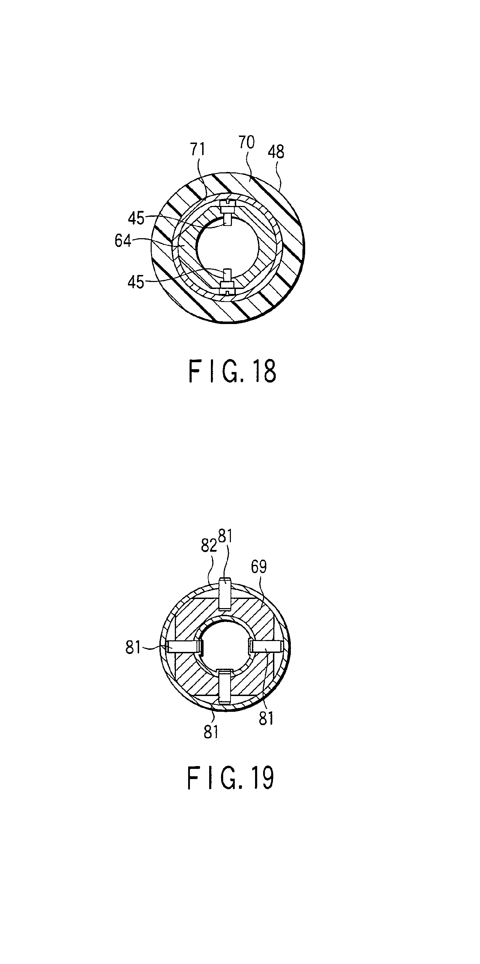

As shown in FIG. 19, four metallic positioning pins 81 are provided in the spring receiver 69 of the spring receiving member 64 to project outward in the radial direction. An engaging hole portions 82 in a long hole shape is formed in the large-diameter portion 73 of the rotation transmission member 71, each pin 81 of the spring receiving member 64 being inserted the corresponding engaging hole portion. The engaging hole portion 82 is provided to be extended in the insertion direction of the probe unit 3. Accordingly, the pin 81 moves along the engaging hole portion 82 when the movable handle 49 is operated. Accordingly, transmission of an advancing/retreating action of the spring receiving member 64 to the rotation transmission member 71 is prevented.

In contrast, when the rotation operation knob 50 is operated to rotate, the rotating action of the rotation transmission member 71 rotating together with the rotation operation knob 50 is transmitted to a spring receiving member 64 side via the pin 81. Accordingly, when the rotation operation knob 50 is operated to rotate, an assembly unit of the rotation transmission member 71, the pin 81, the spring receiving member 64, the slider member 65, and the coil spring 67 provided inside the holding cylinder 48 are driven to integrally rotate in the direction around the axis.

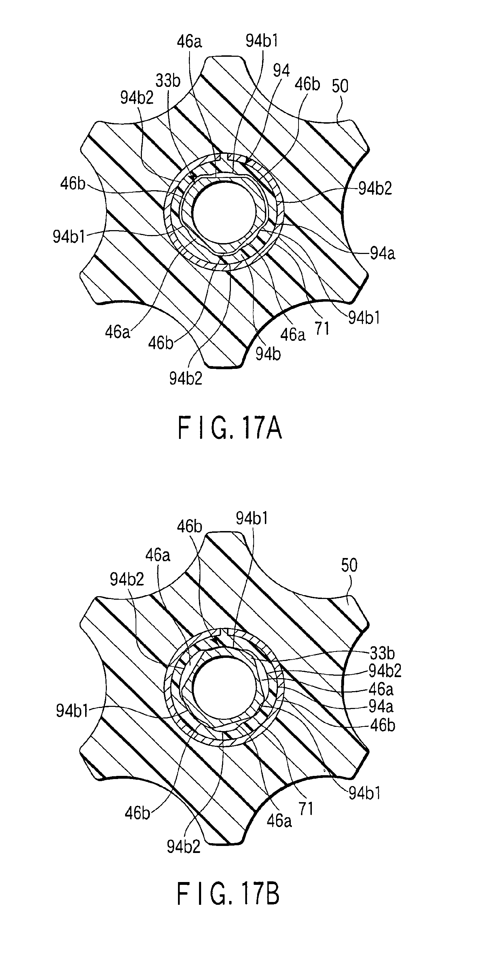

An engaging unit 94 engaged with the outer circumferential flange portion 33b of the sheath unit 5 in a manner allowing to disengage is provided in a substantially central position in the axial direction of the inner circumferential surface of the rotation transmission member 71. As shown in FIGS. 17A and 17B, the engaging unit 94 includes an insertion hole portion 94a into which the outer circumferential flange portion 33b is inserted when the sheath unit 5 and the handle unit 4 are connected, and a conductive rubber ring (energizing unit) 94b arranged inside the insertion hole portion 94a.

A shape of an inner circumferential surface of the conductive rubber ring 94b is formed to have substantially the same shape as that of the engaging portion 46 of the outer circumferential flange portion 33b. That is, the conductive rubber ring 94b is provided with three flat portions 94b1 formed by notching a plurality of locations (three locations in the present embodiment) in the circumferential direction in a circular shape, and three corner portions 94b2 each of which is arranged in joint portions between the three flat portions 94b1 and which have a diameter larger than that of the flat portions 94b1. Accordingly, a sectional shape of the inner circumferential surface of the conductive rubber ring 94b is formed to have a substantially triangular shape. By adopting the configuration as described above, as shown in FIG. 17A, each of the three corner portions 46b of the outer circumferential flange portion 33b matches corresponding one of the three corner portions 94b2 of the conductive rubber ring 94b when the shape of the inner circumferential surface of the conductive rubber ring 94b and the engaging portion 46 of the outer circumferential flange portion 33b are arranged in corresponding positions. In this case, the conductive rubber ring 94b is held in a non-compressed state, which is a natural state. In contrast, as shown in FIG. 17B, the conductive rubber ring 94b is switched to a pressured contact state, in which the conductive rubber ring 94b is contacted by pressure with the three corner portions 46b of the outer circumferential flange portion 33b, by relatively rotating between the handle unit 4 and the sheath unit 5 in the direction around the center axis of the sheath unit 5. At this point, each of the three corner portions 46b of the outer circumferential flange portion 33b is contacted with corresponding one of the three flat portions 94b1 of the conductive rubber ring 94b, and then he three corner portions 46b are compressed.

In the present embodiment, when the sheath unit 5 and the handle unit 4 are connected, first an insertion operation (see FIGS. 31 and 32) to insert the outer circumferential flange portion 33b of the sheath unit 5 straight into the conductive rubber ring 94b is performed. At this point, as shown in FIG. 17A, the conductive rubber ring 94b is held in a non-compressed state, which is a natural state. The engaging lever 43 on the side of the handle unit 4 is held in a state in which the engaging lever 43 is put on the inclined plane of the guide groove 41 of the knob member 32 of the sheath unit 5. Then, the knob member 32 of the sheath unit 5 is rotated in the direction around the axis with respect to the handle unit 4. Accordingly, as shown in FIGS. 33 and 34, the engaging lever 43 on the side of the handle unit 4 is engaged with the engaging recess 42 provided at one end portion of the guide groove 41 by being inserted thereinto. At this point, as shown in FIG. 17B, the conductive rubber ring 94b is switched to a pressured contact state in which the conductive rubber ring 94b is contacted by pressure with the three corner portions 46b of the outer circumferential flange portion 33b. Accordingly, the sheath-unit-side electric path 40 (formed by the guide cylinder 33, the fixing screw 39, the connecting tube 38, the external cylinder 18, the tip cover 25, the fulcrum pin 27, and the jaw main body 28) and the handle unit-side electric path 95 (formed by an electric contact member 96, the spring receiving member 64, the positioning pin 81, and the rotation transmission member 71) are brought into conduction via the conductive rubber ring 94b. Accordingly, a second high-frequency electric path 97 on which a high-frequency current is transmitted is formed in a connected body of the sheath unit 5 and the handle unit 4.

As shown in FIG. 21, the handle unit 4 includes a tubular member 98 formed of an insulating material to the inner circumferential surface side of the spring receiving member 64. The tubular member 98 is fixed to an inner circumferential surface of the spring receiving member 64. Accordingly, when the probe unit 3 and the handle unit 4 are connected, the first high-frequency electric path 13 and the second high-frequency electric path 97 are insulated by the tubular member 98.

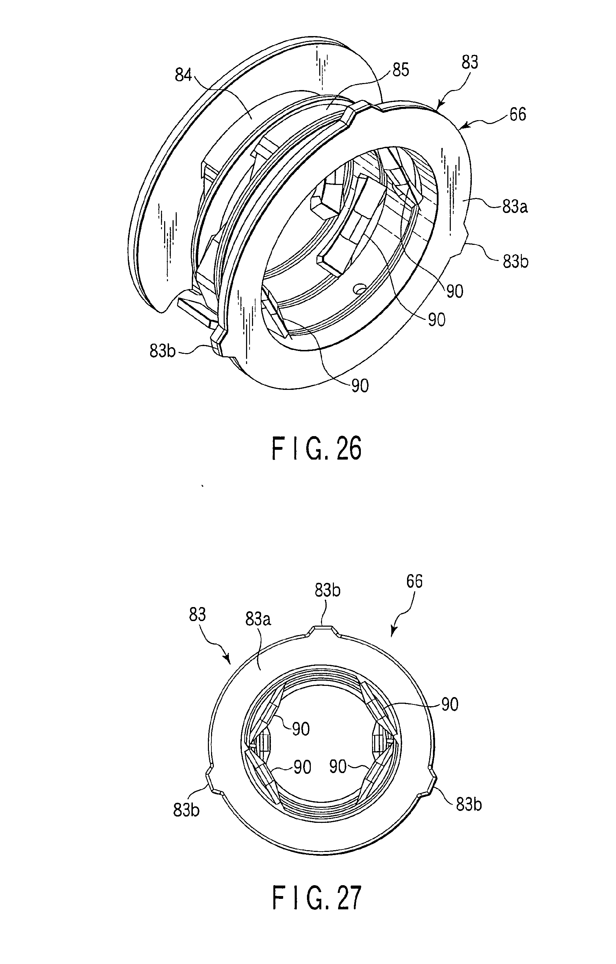

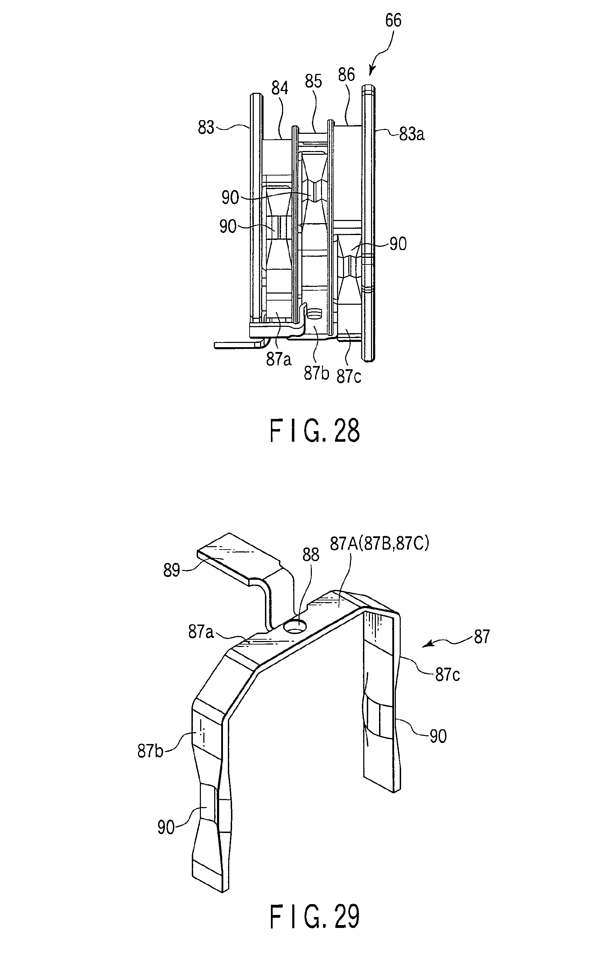

FIGS. 26 to 28 are diagrams showing a contact unit 66 in a cylindrical shape. The distal end portion of the vibrator unit 2 is removably connected to the contact unit 66. The contact unit 66 includes a resin electrode holding member 83 in a cylindrical shape. As shown in FIG. 28, the electrode holding member 83 includes three first to third electrode receiving portions 84, 85, 86 having different outside diameters with respect to each other. The first electrode receiving portion 84 provided on the most distal side has the smallest diameter and the third electrode receiving portion 86 provided on the most proximal side has the largest diameter.

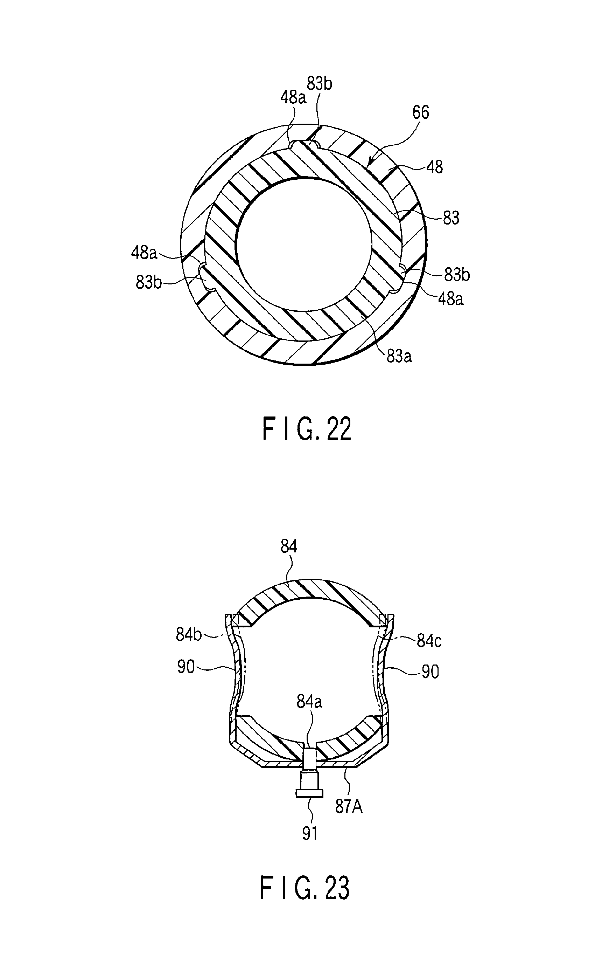

As shown in FIG. 23, the first electrode receiving portion 84 includes one contact member fixing hole 84a and two through holes 84b, 84c. Each of the two through holes 84b, 84c is arranged in position where a center line of each of through holes 84b, 84c is perpendicular to a center line of the contact member fixing hole 84a.

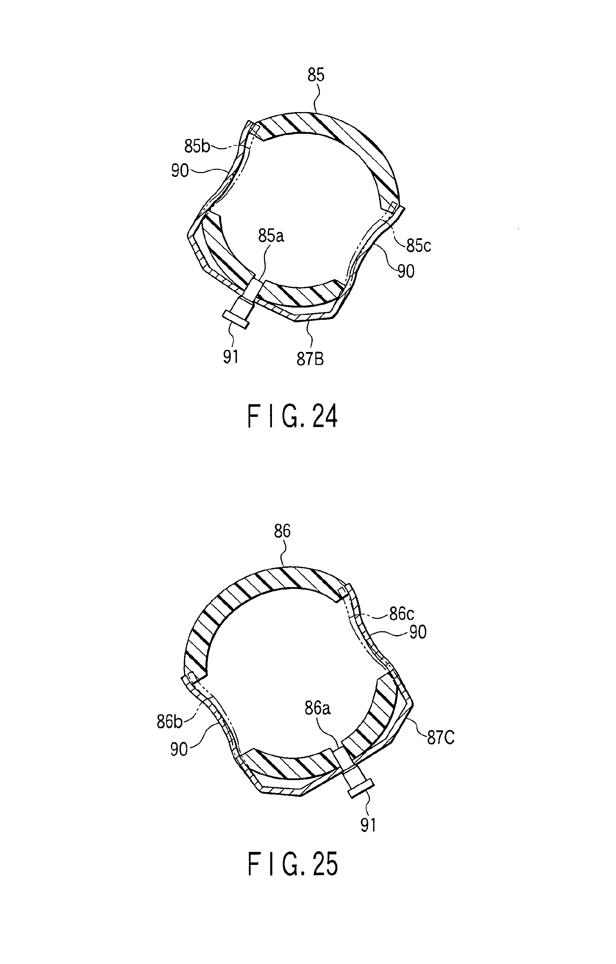

Similarly, as shown in FIG. 24, the second electrode receiving portion 85 includes one contact member fixing hole 85a and two through holes 85b, 85c. As shown in FIG. 25, the third electrode receiving portion 86 includes one contact member fixing hole 86a and two through holes 86b, 86c.

The contact member fixing hole 84a of the first electrode receiving portion 84, the contact member fixing hole 85a of the second electrode receiving portion 85, and the contact member fixing hole 86a of the third electrode receiving portion 86 are arranged with shifted phases in the circumferential direction of the electrode holding member 83 with respect to each other.

FIGS. 29 and 30 show electrode members 87A, 87B, 87C assembled into the first to third electrode receiving portions 84, 85, 86, respectively. The electrode members 87A, 87B, 87C are formed to have the same shape. Here, only the electrode member 87A assembled into the first electrode receiving portion 84 is described and the same portions of the electrode members 87B, 87C, which are assembled into the second and third electrode receiving portions 85, 86, as those of the electrode member 87A are denoted with the same reference numerals to omit a description thereof.

The electrode member 87A includes one linear fixed portion 87a and two bent portions 87b, 87c. The one bent portion 87b is arranged at one end of the linear fixed portion 87a and the other bent portion 87c is arranged at the other end thereof. Accordingly, as shown in FIG. 29, the electrode member 87A is formed by bending into a substantial U shape.

A hole 88 and an electric wire connection portion 89 in an L shape are provided in a central position of the fixed portion 87a. A constricted portion 90 having a shape curved inward toward a central position is formed in each of the two bent portions 87b, 87c.

When the electrode member 87A is assembled into the first electrode receiving portion 84, a fixing pin 91 is inserted into the hole 88 of the fixing portion 87a of the electrode member 87A and the contact member fixing hole 84a of the first electrode receiving portion 84. The electrode member 87A is fixed to the first electrode receiving portion 84 by the fixing pin 91. At this point, the constricted portion 90 of the one bent portion 87b of the electrode member 87A is inserted into the one through hole 85b and the constricted portion 90 of the other bent portion 87c of the electrode member 87A is inserted into the other through hole 85c. This also applies when the electrode member 87B is assembled into the second electrode receiving portion 85 and when the electrode member 87C is assembled into the third electrode receiving portion 86.

As shown in FIG. 22, a large-diameter fixed flange portion 83a is formed in a proximal end portion of the electrode holding member 83 of the contact unit 66. An engaging height 83b is provided on an outer circumferential surface of the fixed flange portion 83a to project at a plurality of locations (three locations in the present embodiment). On the inner circumferential surface of the proximal end portion of the holding cylinder 48, each engaging recess 48a is formed in position corresponding to one of the three engaging heights 83b of the fixed flange portion 83a. When the electrode holding member 83 is assembled into the holding cylinder 48, the electrode holding member 83 is engaged with and fixed to the holding cylinder 48 by each of the three engaging heights 83b of the fixed flange portion 83a being inserted into the corresponding engaging recess 48a of the holding cylinder 48. Accordingly, the rotation of the electrode holding member 83 in the direction around the axis with respect to the holding cylinder 48 is restricted.

As shown in FIGS. 16 and 22, the holding cylinder 48 includes a step portion 48b in contact with the fixed flange portion 83a of the electrode holding member 83 formed therein. The electrode holding member 83 is fixed to the holding cylinder 48 by a fixing screw 48c in a state that the fixed flange portion 83a of the electrode holding member 83 is bumped against the step portion 48b. Accordingly, the movement in the axial direction of the electrode holding member 83 with respect to the holding cylinder 48 is restricted.

An end of each of three electric wires 93a to 93c incorporated into the switch holding section 51 is connected to corresponding one of the three electrode members 87A, 87B, 87C incorporated into the contact unit 66.

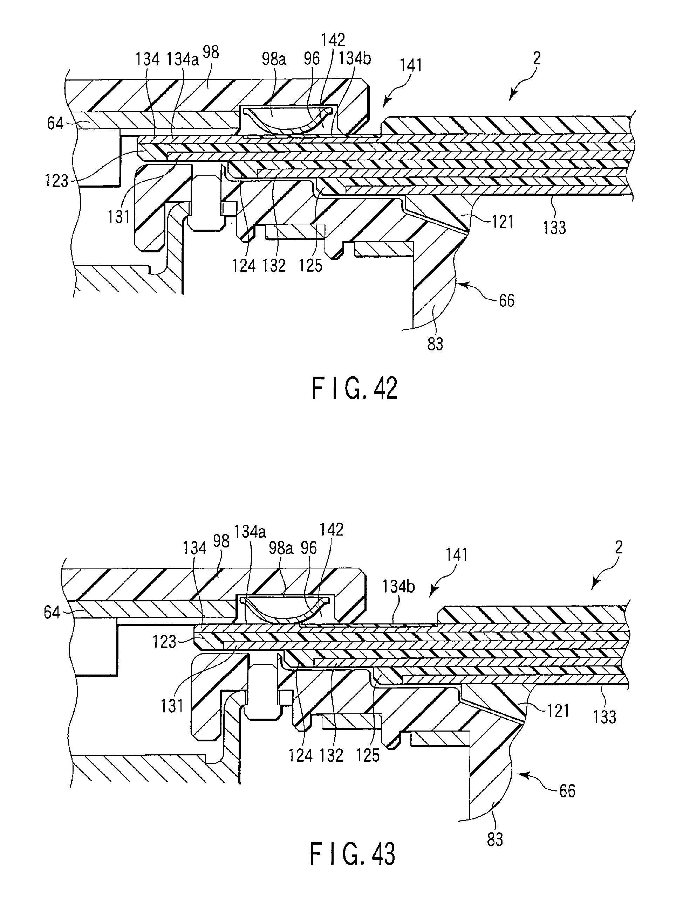

As shown in FIG. 42, an electric contact mounting groove 98a in a depressed shape is formed on an outer circumferential surface of a proximal end portion of the tubular member 98. As shown in FIG. 21, an electric contact member 96 in a substantial C shape formed of a metallic plate spring is mounted in the electric contact mounting groove 98a. The electric contact member 96 is connected to the outer circumferential surface of the proximal end portion of the spring receiving member 64.

Thee engaging heights 99 corresponding to the three engaging recesses 15 (see FIG. 37) of the flange portion 14 of the probe unit 3 are formed on the inner circumferential surface of the tubular member 98. When the probe unit 3 and the handle unit 4 are connected, each of the three engaging heights 99 of the tubular member 98 is engaged with corresponding one of the three engaging recesses 15 of the flange portion 14 of the probe unit 3 in a manner allowing disengagement. Accordingly, the positions of the rotation direction of the probe unit 3 and the tubular member 98 of the handle unit 4 are restricted. Thus, when the rotation operation knob 50 is operated to rotate, a connected body of the probe unit 3 and the vibrator unit 2 is integrally driven to rotate together with the assembled unit inside the holding cylinder 48.

The engaging portion between the flange portion 14 of the probe unit 3 and the tubular member 98 is not limited to the above configuration. For example, the tubular member 98 may be formed to have a D-shaped sectional shape, and the flange portion 14 of the probe unit 3 may be formed in a D-shaped sectional shape corresponding to the tubular member 98.

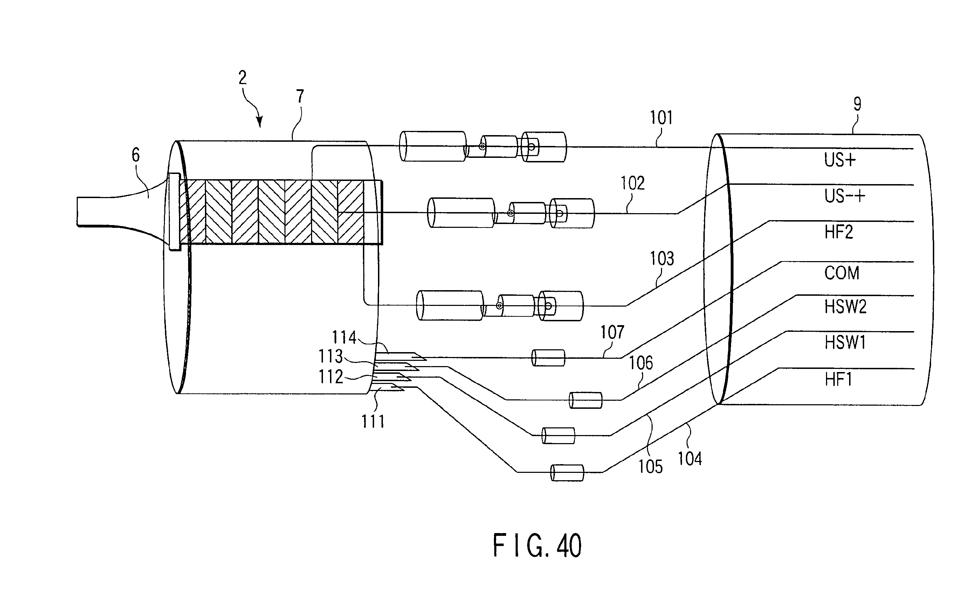

FIG. 40 is a diagram showing a connection state of internal electric wiring in the one cable 9 provided at the proximal end of the vibrator unit 2 and the vibrator unit 2. As shown in FIG. 40, two wires 101, 102 used in the ultrasonic vibrator, two wires 103, 104 used in high-frequency conduction, and three wires 105, 106, 107 connected to the wiring circuit board 92 inside the switch holding section 51 are incorporated into the one cable 9 provided at the proximal end of the vibrator unit 2. Distal end portions of the two wires 101, 102 used in the ultrasonic vibrator are connected to the ultrasonic vibrator 6. A distal end portion of the one wire 103 used in high-frequency conduction is connected to the ultrasonic vibrator 6.

Four first to fourth conductive plates 111 to 114 used in electric connection are disposed at the proximal end of the vibrator unit 2. A distal end portion of the other wire 104 used in high-frequency conduction is connected to the first conductive plate 111. The three wires 105, 106, 107 are connected to the second to fourth conductive plates 112 to 114 respectively.

FIG. 41 is a diagram showing the internal configuration of the distal end portion of the vibrator unit 2. A connecting cylindrical portion 121 is formed in the distal end portion of the vibrator cover 7. A C ring 122 such as a plate spring formed by notching a part of a ring is mounted on an outer circumferential surface of the connecting cylindrical portion 121. First to third cylindrical portions 123 to 125 in three stages having mutually different outside diameter dimensions are provided on the inner side of the connecting cylindrical portion 121 to project. The first cylindrical portion 123 has the smallest outside diameter and the longest projection length from a distal end of the connecting cylindrical portion 121. The second cylindrical portion 124 has a larger outside diameter than the first cylindrical portion 123 and a shorter projection length from the distal end of the connecting cylindrical portion 121 than the first cylindrical portion 123. The third cylindrical portion 125 has the largest outside diameter and a shorter projection length from the distal end of the connecting cylindrical portion 121 than the second cylindrical portion 124.

A first contact member 131 in a cylindrical shape is mounted on an outer circumferential surface of the first cylindrical portion 123. Similarly, a second contact member 132 in a cylindrical shape is mounted on an outer circumferential surface of the second cylindrical portion 124 and a third contact member 133 in a cylindrical shape is mounted on an outer circumferential surface of the third cylindrical portion 125. The second conductive plate 112 is connected to the first contact member 131, the third conductive plate 113 is connected to the second contact member 132, and the fourth conductive plate 114 is connected to the third contact member 133.

A fourth contact member 134 in a cylindrical shape is mounted on an inner circumferential surface of the first cylindrical portion 123. The fourth contact member 134 is connected to the first conductive plate 111.

When the handle unit 4 and the vibrator unit 2 are connected, the contact unit 66 of the handle unit 4 and the distal end portion of the vibrator unit 2 are connected. At this point, the electrode member 87A of the contact unit 66 and the first contact member 131 of the vibrator unit 2 are connected. At the same time, the electrode member 87B of the contact unit 66 and the second contact member 132 of the vibrator unit 2, the electrode member 87C of the contact unit 66 and the third contact member 133 of the vibrator unit 2, and the C-shaped electric contact member 96 of the tubular member 98 and the fourth contact member 134 of the vibrator unit 2 are connected, respectively.

Further, the handpiece 1 in the present embodiment includes an output mode switching section (output mode switching mechanism) 141 (see FIGS. 42, 43). The output mode switching section 141 is a switching mechanism configured to selectively switch the output mode of the surgical apparatus between a bipolar mode in which the probe distal end 11a and the jaw 17 are driven as high-frequency electrodes and a probe single output mode in which energy is output only from the probe distal end 11a.

The output mode switching section 141 is composed of any movable member / movable members of operation force transmission members that are driven to advance or retreat in the axial direction of the probe 11 in accordance with an operation of the movable handle 49 and transmit an operation force of the movable handle 49 to the jaw 17. That is, the output mode switching section 141 is composed of a part of the slider member 65, the coil spring 67, the spring receiving member 64, the tubular member 98, the connecting capsule 34 and the like. In the present embodiment, the output mode switching section 141 constituted as described below is provided in a connection portion between the C-shaped electric contact member 96 of the tubular member 98 of the handle unit 4 and the fourth contact member 134 of the vibrator unit 2.

As shown in FIG. 42, a conductive portion 134a whose metal material is exposed and an insulated portion 134b obtained by an insulation coating on the surface of the metal material are formed on a surface (inner circumferential surface) of the fourth contact member 134. The insulated portion 134b is formed of, for example, a fluorine base coating, DLC coating or the like. The conductive portion 134a is arranged on a distal end side site of the fourth contact member 134 and the insulated portion 134b is arranged on a proximal end side site of the conductive portion 134a. When the movable handle 49 is operated, the tubular member 98 moves in the axial direction of the probe 11. With the movement of the tubular member 98, the contact part (one of the conductive portion 134a and the insulated portion 134b of the fourth contact member 134) of the fourth contact member 134 in contact with the C-shaped electric contact member 96 is switched. In this case, the state in which the conductive portion 134a of the fourth contact member 134 is in contact with the C-shaped electric contact member 96 and the state in which the insulated portion 134b of the fourth contact member 134 is in contact with the C-shaped electric contact member 96 are switched. Accordingly, a contact switching section 142 configured to perform an opening/closing operation of a contact, which is configured to connect or cut off the conduction state of a high-frequency current to the jaw 17, is formed.

FIG. 42 is a diagram showing a moving state of the output mode switching section 141 in a state that the jaw 17 is open. That is, FIG. 42 is a diagram in which the movable handle 49 is operated in a direction away from the fixed handle 47 and the tubular member 98 moves to the proximal end side. At this point, the C-shaped electric contact member 96 of the tubular member 98 is in contact with the insulated portion 134b of the fourth contact member 134 of the vibrator unit 2. Thus, an electric connection between the C-shaped electric contact member 96 of the tubular member 98 and the fourth contact member 134 of the vibrator unit 2 is cut off. Therefore, conduction of a high-frequency current to the jaw 17 is cut off. Consequently, the output mode switching section 141 is switched to the probe single output mode. In probe single output mode, ultrasonic waves only from the vibrator 6 are output to the probe 11.

FIG. 43 is a diagram showing the moving state of the output mode switching section 141 in a state that the jaw 17 is closed. That is, FIG. 43 is a diagram in which the movable handle 49 is operated in a direction toward the fixed handle 47 and the tubular member 98 moves to the distal end side. At this point, the C-shaped electric contact member 96 of the tubular member 98 is in contact with the conductive portion 134a of the fourth contact member 134 of the vibrator unit 2. Thus, the C-shaped electric contact member 96 of the tubular member 98 and the fourth contact member 134 of the vibrator unit 2 are electrically connected and in conduction. Therefore, the output mode switching section 141 is switched to the bipolar mode. In the bipolar mode, a high-frequency current is supplied to the jaw 17. In the handpiece 1 of the ultrasonic treatment device, the probe 11 and the jaw 17 are driven as high-frequency electrodes respectively. In this case, the probe 11 may be configured to output ultrasonic waves as energy at the same time.

Next, functions of the present embodiment will be described. As shown in FIG. 2, in the handpiece 1 of an ultrasonic treatment device according to the first embodiment, each of four units of the vibrator unit 2, the probe unit 3, the handle unit 4, and the sheath unit 5 can be removed. When the handpiece 1 is used, the vibrator unit 2 and the probe unit 3 are connected. Accordingly, the first high-frequency electric path 13 on which a high-frequency current can be transmitted is formed in a connected body of the vibrator unit 2 and the probe unit 3 is formed.

Then, the handle unit 4 and the sheath unit 5 are connected. When the handle unit 4 and the sheath unit 5 are connected, the connecting capsule 34 is inserted into the rotation transmission member 71 of the handle unit 4 in the state that the knob member 32 of the sheath unit 5 is gripped. As shown in FIGS. 31 and 32, when the sheath unit 5 and the handle unit 4 are connected, the engaging lever 43 provided to the side of the handle unit 4 is held in a state in which the engaging lever 43 is put on the inclined plane of the guide groove 41 of the knob member 32 of the sheath unit 5. At this point, as shown in FIG. 17A, the shape of the inner circumferential surface of the conductive rubber ring 94b and the engaging portion 46 of the outer circumferential flange portion 33b are held in corresponding positions, that is, in a state in which each of the three corner portions 46b of the outer circumferential flange portion 33b matches corresponding one of the three corner portions 94b2 of the conductive rubber ring 94b. Thus, the outer circumferential flange portion 33b of the sheath unit 5 is inserted straight into the conductive rubber ring 94b. As shown in FIG. 17A, the conductive rubber ring 94b is held in a non-compressed state position, which is a natural state, during an insertion operation. At this point, the sheath-unit-side electric path 40 and the handle unit-side electric path 95 are not in conduction.

Then, after the insertion operation is completed, an operation to rotate the knob member 32 of the sheath unit 5 in the direction around the axis with respect to the handle unit 4 is performed. With the rotation operation, as shown in FIGS. 33 and 34, the engaging lever 43 provided to the side of the handle unit 4 is engaged with the engaging recess 42 provided at one end of the guide groove 41 by being inserted thereinto. At this point, as shown in FIG. 17B, the conductive rubber ring 94b is switched to a pressured contact state in which the conductive rubber ring 94b contacted by pressure to the three corner portions 46b of the outer circumferential flange portion 33b. Accordingly, the sheath-unit-side electric path 40 and the handle unit-side electric path 95 are brought into conduction via the conductive rubber ring 94b. Therefore, the second high-frequency electric path 97 on which a high-frequency current can be transmitted is formed in a connected body of the sheath unit 5 and the handle unit 4.

When the sheath unit 5 is operated to rotate in the direction around the axis, at the same time, the pair of engaging pins 45 provided to the side of the handle unit 4 are engaged with the engaging groove 44a provided in the termination portion (proximal end portion) of the guide groove 44 of the sheath unit 5 in a manner allowing disengagement. Accordingly, the spring receiving member 64 provided to the side of the handle unit 4 and the connecting capsule 34 provided to the side of the sheath unit 5 are connected via the engaging pin 45. Accordingly, the operation force, which act to the side of the handle unit 4 when the movable handle 49 is operated to close with respect to the fixed handle 47, is made transmittable to the drive shaft 21 of the jaw 17 provided to the side of the sheath unit 5. This state is the connected state of the sheath unit 5 and the handle unit 4.

Then, the connected body of the sheath unit 5 and the handle unit 4 and the connected body of the ultrasonic vibrator 6 and the probe unit 3 are assembled to be combined. For the assembly, the contact unit 66 of the handle unit 4 and the distal end portion of the vibrator unit 2 are connected. At this point, the electrode member 87A of the contact unit 66 and the first contact member 131 of the vibrator unit 2 are connected. At the same time, the electrode member 87B of the contact unit 66 and the second contact member 132 of the vibrator unit 2, the electrode member 87C of the contact unit 66 and the third contact member 133 of the vibrator unit 2, and the C-shaped electric contact member 96 of the tubular member 98 and the fourth contact member 134 of the vibrator unit 2 are connected. Accordingly, the second high-frequency electric path 97 of the connected body of the sheath unit 5 and the handle unit 4 is connected to the wire 104 used in high-frequency conduction and provided inside the cable 9. Further, the three wires 105, 106, 107 inside the cable 9 are connected to the wiring circuit board 92 inside the switch holding section 51. This state is a termination state of assembly of the handpiece 1.

When the handpiece 1 is used, the drive shaft 21 is moved in the axial direction in accordance with the operation of the movable handle 4 by performing an opening/closing operation of the movable handle 49 with respect to the fixed handle 47. The jaw 17 is driven to open/close with respect to the probe distal end 11a of the probe unit 3 by being linked to the advancing/retreating movement of the drive shaft 21 in the axial direction.

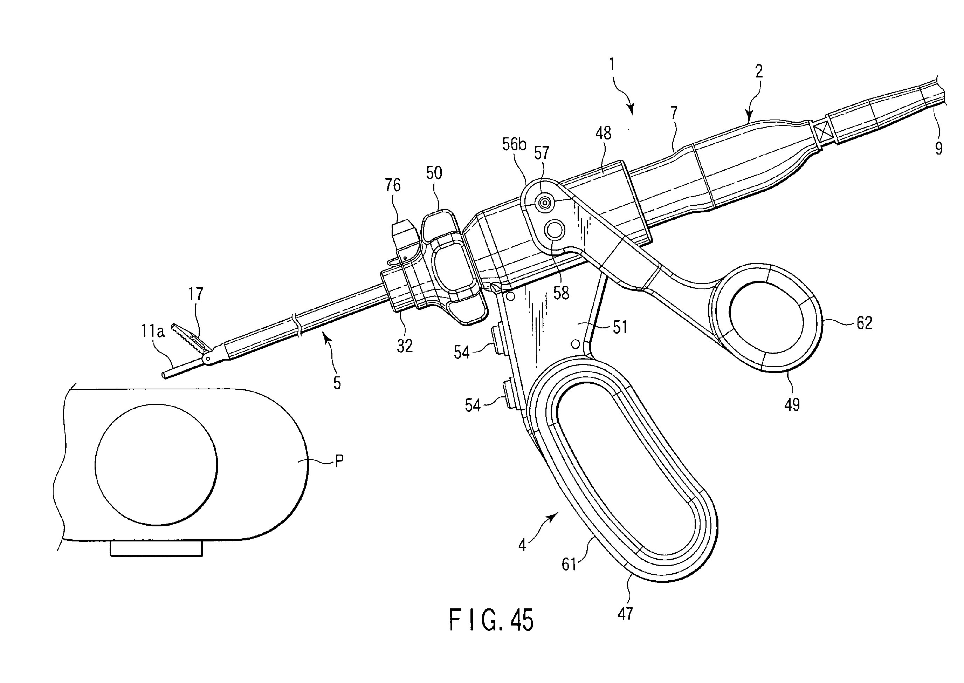

If the movable handle 49 is operated in the direction away from the fixed handle 47 (opening operation), the drive shaft 21 is towed to the proximal end side in accordance with the operation of the movable handle 49. Thus, the jaw 17 is operated to open. At this point, the output mode switching section 141 is switched to the probe single output mode shown in FIG. 42. In this state, the C-shaped electric contact member 96 of the tubular member 98 is in contact with the insulated portion 134b of the fourth contact member 134 of the vibrator unit 2 and thus, an electric connection between the C-shaped electric contact member 96 of the tubular member 98 and the fourth contact member 134 of the vibrator unit 2 is cut off. Accordingly, conduction of a high-frequency current to the jaw 17 is cut off in the probe single output mode and thus, ultrasonic waves only from the vibrator 6 are output to the probe 11. Therefore, as shown in FIG. 45, a patient P is treated with ultrasonic vibrations only output from the probe distal end 11a in the probe single output mode. In this state, for example, treatment such as a puncture is possible by using cavitation of the probe 11.

If the movable handle 49 is operated in the direction toward the fixed handle 47 (closing operation), the drive shaft 21 is pushed to the distal end side in accordance with the operation of the movable handle 49, and then the jaw 17 is moved in the closing direction. Thus, it becomes possible to grip tissues between the probe distal end 11a and the jaw 17. At this point, the output mode switching section 141 is switched to the bipolar mode shown in FIG. 43. In this state, the C-shaped electric contact member 96 of the tubular member 98 is in contact with the conductive portion 134a of the fourth contact member 134 of the vibrator unit 2 and thus, the C-shaped electric contact member 96 of the tubular member 98 and the fourth contact member 134 of the vibrator unit 2 are electrically connected and in conduction. Accordingly, a high-frequency current is supplied to the jaw 17 in the bipolar mode. In this state, the handpiece 1 of the ultrasonic treatment device is driven in bipolar mode in which each of the probe 11 and the jaw 17 becomes a high-frequency electrode. At this point, strong coagulation and quick incision of tissues, provided between the probe distal end 11a and jaw 17, become possible by simultaneously outputting ultrasonic waves as energy from the probe 11.

Therefore, the following effects are gained from the above configuration. That is, the handpiece 1 of an ultrasonic treatment device in the present embodiment is provided with the output mode switching section 141 and thus, the output of a surgical apparatus can selectively be switched between the bipolar mode in which the probe distal end 11a and the jaw 17 are driven as high-frequency electrodes and the probe single output mode in which energy is output only from the probe distal end 11a in accordance with the operation of the movable handle 49. If the movable handle 49 is operated in the direction away from the fixed handle 47 (opening operation), the output mode switching section 141 is switched to the probe single output mode shown in FIG. 42. In the present embodiment, conduction of a high-frequency current to the jaw 17 is cut off in this state and thus, ultrasonic waves only from the vibrator 6 are output to the probe 11. Therefore, as shown in FIG. 45, the patient P is treated with ultrasonic vibrations only output from the probe distal end 11a in the probe single output mode. In this state, for example, treatment such as a puncture is possible by using cavitation of the probe 11.

Further, if the movable handle 49 is operated in the direction toward the fixed handle 47 (closing operation), the output mode switching section 141 is switched to the bipolar mode shown in FIG. 43. In the bipolar mode, a high-frequency current is supplied to the jaw 17. In this state, each of the probe 11 and the jaw 17 of the handpiece 1 of the ultrasonic treatment device is driven as a high-frequency electrode. At this point, strong coagulation and quick incision of tissues, provided between the probe distal end 11a and jaw 17, become possible by simultaneously outputting ultrasonic waves as energy from the probe 11.

Therefore, according to the handpiece 1 of an ultrasonic treatment device in the present embodiment, the bipolar mode and the probe single output mode can selectively be switched easily by operating the movable handle 49 in accordance with an appropriate situation used in treatment even if only one unit of the handpiece 1 is used in treatment. Consequently, troublesome operations such as an operation to continue work by interchanging a monopolar treatment apparatus and a bipolar treatment apparatus become unnecessary. Accordingly, compared with a case when work is continued by interchanging a monopolar treatment apparatus and a bipolar treatment apparatus, operability of operation by the user is improved and the treatment time of the whole surgery can be reduced.

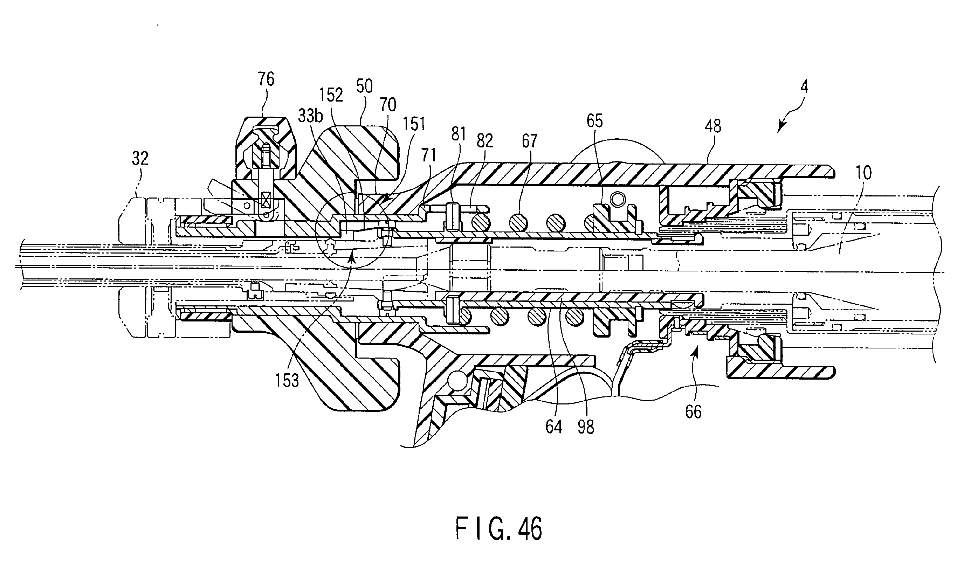

FIGS. 46 to 48 are diagrams showing the second embodiment of the present embodiment. FIG. 46 is a longitudinal sectional view showing an internal configuration of a handpiece 1 of an ultrasonic treatment device in the present embodiment. In the present embodiment, an output mode switching section 151 constituted differently from the handpiece 1 in the first embodiment (see FIGS. 1 to 45) is provided. The configuration other than this changed portion (mode switching section 151) is substantially the same as that of the first embodiment (see FIGS. 1 to 45). Thus, in FIGS. 46 to 48, the same reference numerals are attached to the same portions as those in the first embodiment and a description thereof is omitted.

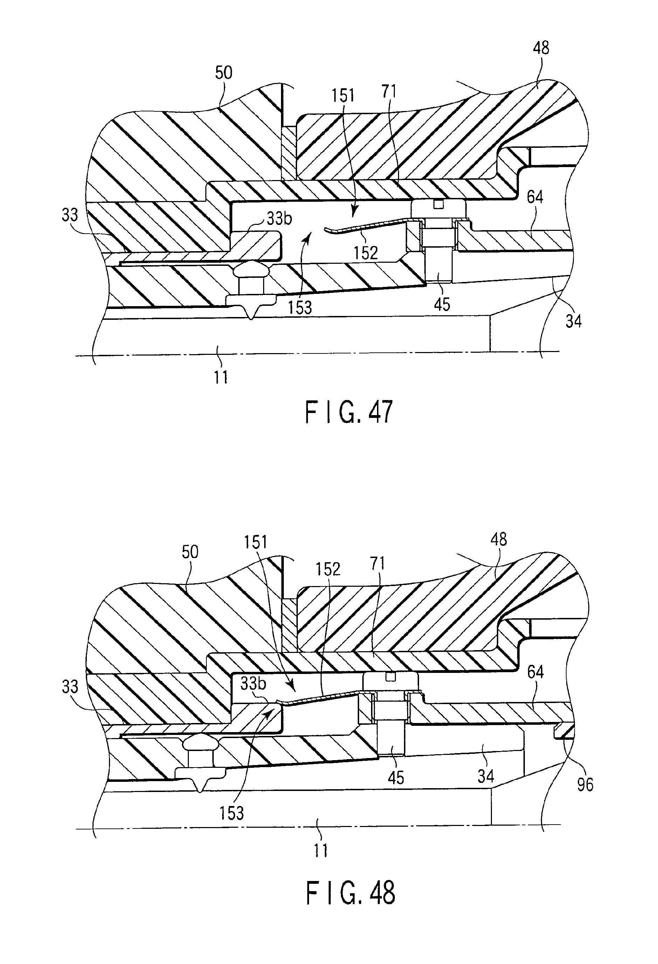

As shown in FIGS. 47 and 48, the output mode switching section 151 in the present embodiment is provided between the spring receiving member 64 provided to the side of the handle unit 4 and the outer circumferential flange portion 33b of the guide cylinder 33 of the sheath unit 5. A contact member 152 of a metal plate extended toward the distal end side is mounted on the distal end portion of the spring receiving member 64 via the engaging pin 45. When the movable handle 49 is operated, the contact member 152 comes into contact with the outer circumferential flange portion 33b of the guide cylinder 33 of the sheath main body 16 in a manner allowing contact and separation.

When the movable handle 49 is operated, the spring receiving member 64 moves in the axial direction of the probe 11. The action of the spring receiving member 64 causes switching to one of the state in which the contact member 152 of the spring receiving member 64 and the outer circumferential flange portion 33b of the guide cylinder 33 are in contact as shown in FIG. 48 and the state in which the contact member 152 of the spring receiving member 64 and the outer circumferential flange portion 33b of the guide cylinder 33 are not in contact as shown in FIG. 47. That is, the contact member 152 forms a contact switching section 153 which is configured to perform an opening/closing operation of the contact to connect or cut off the conduction state of a high-frequency current to the jaw 17 in accordance with the action of the spring receiving member 64 in response to an operation of the movable handle 49. In the present embodiment, the rotation transmission member 71 provided in the distal end portion of the holding cylinder 48 is formed of an insulating resin material.

FIG. 47 is a diagram showing the moving state of the output mode switching section 151 in a state that the jaw 17 is opened. That is, FIG. 47 is a diagram in which the movable handle 49 is operated in the direction away from the fixed handle 47 and the spring receiving member 64 moves to the proximal end side. At this point, the contact member 152 of the spring receiving member 64 is apart from the outer circumferential flange portion 33b of the guide cylinder 33. Thus, an electric connection between the contact member 152 of the spring receiving member 64 and the outer circumferential flange portion 33b of the guide cylinder 33 is cut off. In this state, conduction of a high-frequency current to the jaw 17 is cut off. Thus, the output mode switching section 151 is switched to the probe single output mode. In the probe single output mode, ultrasonic waves only from the vibrator 6 are output to the probe 11.

FIG. 48 is a diagram showing the moving state of the output mode switching section 151 in a state that the jaw 17 is closed. That is, FIG. 48 is a diagram in which the movable handle 49 is operated in the direction toward the fixed handle 47 and the spring receiving member 64 moves to the distal end side. At this point, the contact member 152 of the spring receiving member 64 is in contact with the outer circumferential flange portion 33b of the guide cylinder 33. Thus, the contact member 152 of the spring receiving member 64 and the outer circumferential flange portion 33b of the guide cylinder 33 are electrically connected and in conduction. In this state, the output mode switching section 151 is switched to the bipolar mode. In the bipolar mode, a high-frequency current is supplied to the jaw 17. In this state, each of the probe 11 and the jaw 17 of the handpiece 1 of the ultrasonic treatment device is driven as a high-frequency electrode. In this case, ultrasonic waves may be output as energy from the probe 11 at the same time.

Therefore, the following effects are gained from the above configuration. That is, the handpiece 1 of an ultrasonic treatment device in the present embodiment is provided with the output mode switching section 151 and thus, the output of a surgical apparatus can be selectively switched between the bipolar mode in which the probe distal end 11a and the jaw 17 are driven as high-frequency electrodes and the probe single output mode in which energy is output only from the probe distal end 11a in accordance with the operation of the movable handle 49. If the movable handle 49 is operated in a direction away from the fixed handle 47 (opening operation), the output mode switching section 151 is switched to the probe single output mode shown in FIG. 47. In the present embodiment, conduction of a high-frequency current to the jaw 17 is cut off in this state and thus, ultrasonic waves only from the vibrator 6 are output to the probe 11. Therefore, as shown in FIG. 45, the patient P is treated with ultrasonic vibrations only output from the probe distal end 11a in probe single output mode. In this state, for example, treatment such as a puncture is possible by using cavitation of the probe 11.

Further, if the movable handle 49 is operated in a direction toward the fixed handle 47 (closing operation), the output mode switching section 151 is switched to the bipolar mode shown in FIG. 48. In bipolar mode, a high-frequency current is supplied to the jaw 17. In this state, each of the probe 11 and the jaw 17 of the handpiece 1 of the ultrasonic treatment device is driven as a high-frequency electrode. At this point, strong coagulation and quick incision of tissues, provided between the probe tip 11a and jaw 17, become possible by simultaneously outputting ultrasonic waves as energy from the probe 11.

Therefore, according to the handpiece 1 of an ultrasonic treatment device in the present embodiment, the bipolar mode and the probe single output mode can be selectively switched easily by operating the movable handle 49 in accordance with the appropriate situation used in treatment even if only one unit of the handpiece 1 is used in treatment. Consequently, troublesome operations such as an operation to continue work by interchanging a monopolar treatment apparatus and a bipolar treatment apparatus become unnecessary. Accordingly, also in the present embodiment like in the first embodiment, compared with a case when work is continued by interchanging a monopolar treatment apparatus and a bipolar treatment apparatus, operability of operation by the user is improved and the treatment time of the whole surgery can be reduced.

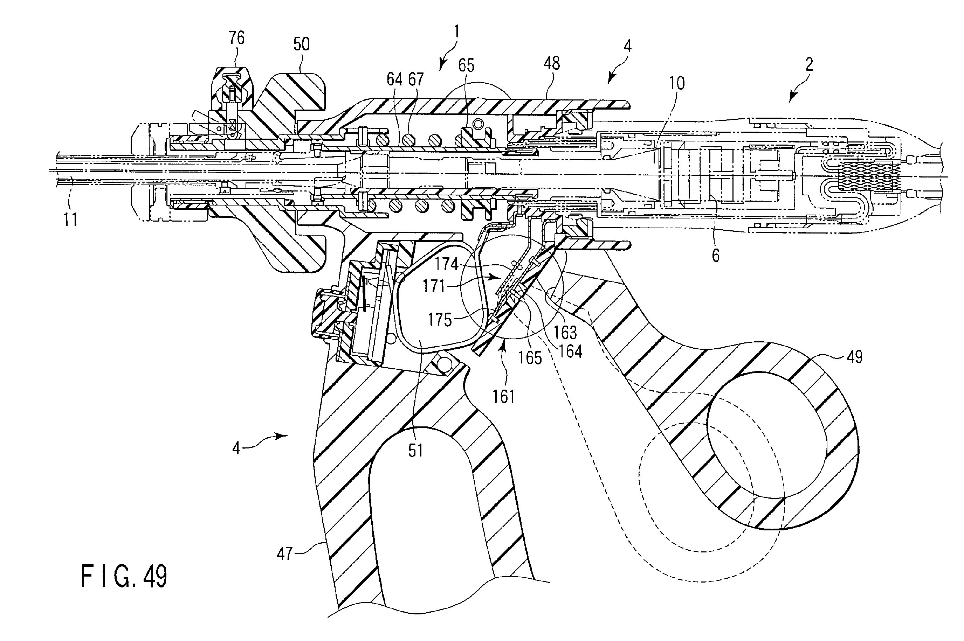

FIGS. 49 to 51 are diagrams showing the third embodiment of the present embodiment. FIG. 49 is a longitudinal sectional view showing an internal configuration of a handpiece 1 of an ultrasonic treatment apparatus in the present embodiment. In the present embodiment, an output mode switching section 161 constituted differently from the handpiece 1 in the first embodiment (see FIGS. 1 to 45) is provided. The configuration other than this changed portion (mode switching section 161) is substantially the same as that of the first embodiment (see FIGS. 1 to 45). Thus, in FIGS. 49 to 51, the same reference numerals are attached to the same portions as those in the first embodiment and a description thereof is omitted.

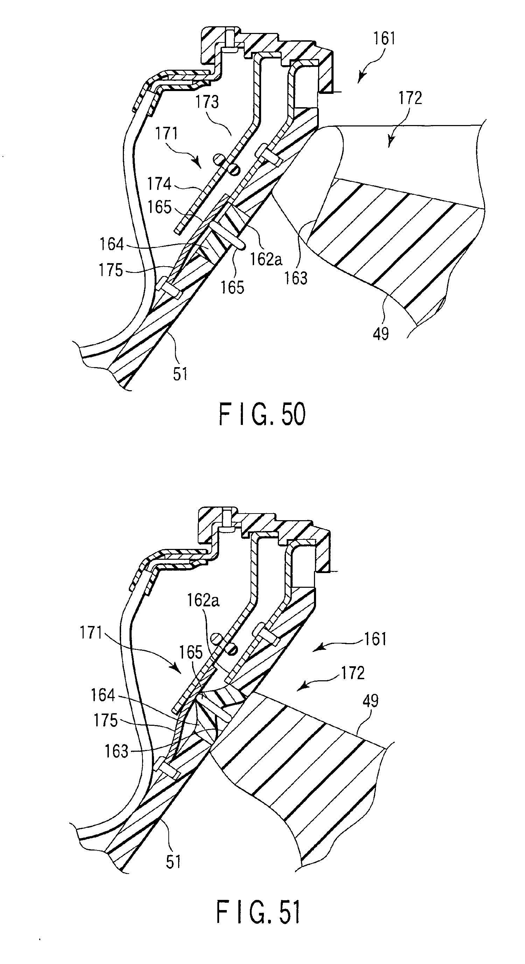

As shown in FIGS. 50 and 51, the output mode switching section 161 in the present embodiment includes a contact switching section 171 which is mounted inside a wall portion 162 provided on a rear surface side (proximal end side surface) of the switch holding section 51 of the handle unit 4, and a contact operation section 172 configured to operate the contact switching section 171.

The contact switching section 171 includes a switching member 173 installed on a midway of the conduction path of a high-frequency current to the jaw 17. The switching member 173 includes a fixed contact member 174 and a movable contact member 175 capable of coming into contact with and separating from the fixed contact member 174. One end of the movable contact member 175 is fixed to an inner surface of the wall portion 162 provided on the rear surface side of the switch holding unit 51. The other end of the movable contact member 175 is held in a position apart from the fixed contact member 174.

An opening 162a is formed in a position corresponding to the other end of the movable contact member 175 in the wall portion 162 provided on the rear surface side of the switch holding section 51. An elastic deformation portion 164 such as an elastically deformable rubber plate is provided in the opening 162a. A pressing pin 165 projects from the elastic deformation portion 164.

The movable handle 49 includes a pressing portion 163 which is configured to press the pressing pin 165 of the elastic deformation portion 164 of the switch holding section 51 and which is provided in a connecting portion between the two arms 56a, 56b. When the movable handle 49 is operated to close, the other end of the movable contact member 175 is pressed in a direction to contact by pressure the fixed contact member 174 by the pressing pin 165 being pressed by the pressing portion 163 of the movable handle 49. Accordingly, the switching member 173 of the contact switching section 171 is actuated to perform an opening/closing operation of the contact which is configured to connect or cut off the conduction state of a high-frequency current to the jaw 17.

When the movable handle 49 is operated, the pressing portion 163 of the movable handle 49 moves in a direction toward or away from the pressing pin 165. The action of the pressing portion 163 causes switching to one of the state in which the pressing portion 163 presses the pressing pin 165 to press the other end of the movable contact member 175 in a direction to contact by pressure the fixed contact member 174 as shown in FIG. 51 and the non-contact state in which the pressing portion 163 moves away from the pressing pin 165 to move the other end of the movable contact member 175 away from the fixed contact member 174 as shown in FIG. 50.

FIG. 50 is a diagram showing the moving state of the output mode switching section 161 in a state that the jaw 17 is opened. That is, FIG. 50 is a diagram showing a non-contact state in which the movable handle 49 is operated in a direction away from the fixed handle 47 and the pressing portion 163 of the movable handle 49 moves away from the pressing pin 165 to separate the other end of the movable contact member 175 from the fixed contact member 174. At this point, the switching member 173 is held in the open state and thus, conduction of a high-frequency current to the jaw 17 is cut off. Therefore, the output mode switching section 161 is switched to the probe single output mode. In the probe single output mode, ultrasonic waves only from the vibrator 6 are output to the probe 11.

FIG. 51 is a diagram showing the moving state of the output mode switching section 161 in a state that the jaw 17 is closed. That is, FIG. 51 is a diagram showing a state in which the movable handle 49 is operated in a direction toward the fixed handle 47 and the other end of the movable contact member 175 is pressed in a direction to contact by pressure the fixed contact member 174 by the pressing pin 165 being pressed by the pressing portion 163 of the movable handle 49. At this point, the switching member 173 is operated to close and a conduction path of a high-frequency current to the jaw 17 is in conduction. In this state, the output mode switching section 161 is switched to the bipolar mode. In bipolar mode, a high-frequency current is supplied to the jaw 17. In this state, each of the probe 11 and the jaw 17 of the handpiece 1 of the ultrasonic treatment device is driven as a high-frequency electrode. In this case, the probe 11 may be configured to output ultrasonic waves as energy at the same time.

Therefore, the following effects are gained from the above configuration. That is, the handpiece 1 of an ultrasonic treatment device in the present embodiment is provided with the output mode switching section 161 and thus, the output of a surgical apparatus is selectively switched between the bipolar mode in which the probe tip 11a and the jaw 17 are driven as high-frequency electrodes and the probe single output mode in which energy is output only from the probe tip 11a in accordance with the operation of the movable handle 49. If the movable handle 49 is operated in a direction away from the fixed handle 47 (opening operation), the output mode switching section 161 is switched to the probe single output mode shown in FIG. 50. In the present embodiment, conduction of a high-frequency current to the jaw 17 is cut off in the probe single output mode and thus, and ultrasonic waves only from the vibrator 6 are output to the probe 11. Therefore, as shown in FIG. 45, the patient P is treated with ultrasonic vibrations only output from the probe distal end 11a in the probe single output mode. In this state, for example, treatment such as a puncture is possible by using cavitation of the probe 11.

Further, if the movable handle 49 is operated in a direction toward the fixed handle 47 (closing operation), the output mode switching section 161 is switched to the bipolar mode shown in FIG. 51. In the bipolar mode, a high-frequency current is supplied to the jaw 17. In this state, each of the probe 11 and the jaw 17 of the handpiece 1 of the ultrasonic treatment device is driven as a high-frequency electrode. At this point, strong coagulation and quick incision of tissues, provided between the probe distal end 11a and jaw 17, become possible by simultaneously outputting ultrasonic waves as energy from the probe 11.

Therefore, according to the handpiece 1 of an ultrasonic treatment device in the present embodiment, the bipolar mode and the probe single output mode can be selectively switched easily by operating the movable handle 49 to perform an appropriate treatment. Consequently, troublesome operations such as an operation to continue work by interchanging a monopolar treatment apparatus and a bipolar treatment apparatus become unnecessary. Accordingly, also in the present embodiment like in the first embodiment, compared with a case when work is continued by interchanging a monopolar treatment apparatus and a bipolar treatment apparatus, operability of operation by the user is improved and the treatment time of the whole surgery can be reduced.

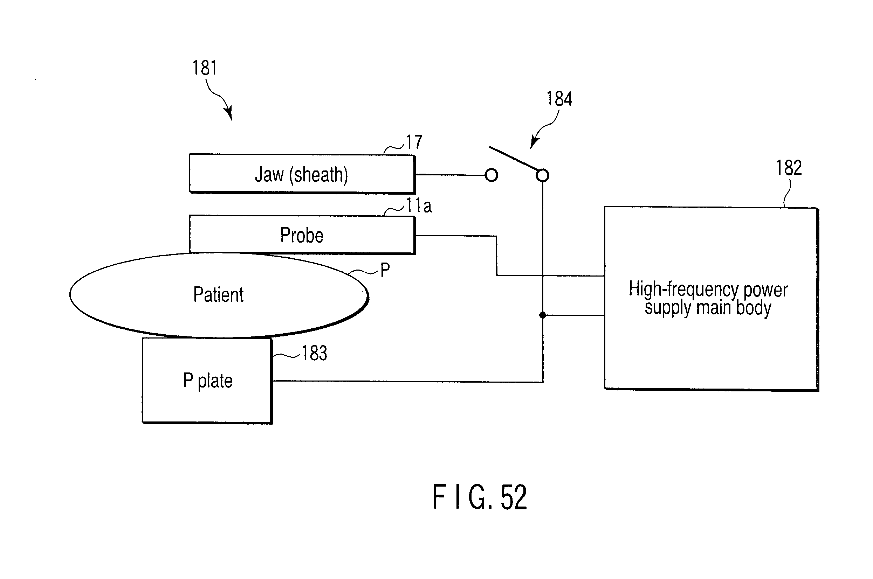

FIG. 52 is a diagram showing the fourth embodiment of the present invention. FIG. 52 is a block diagram schematically showing an electric circuit of a whole system of an ultrasonic treatment device according to the present embodiment. In the present embodiment, a surgical apparatus 181 constituted differently from the system of an ultrasonic treatment device in the first embodiment (see FIGS. 1 to 45) is provided. The configuration other than this changed portion (surgical apparatus 181) is substantially the same as that of the first embodiment (see FIGS. 1 to 45). Thus, in FIG. 52, the same reference numerals are attached to the same portions as those in the first embodiment and a description thereof is omitted.

The surgical apparatus 181 in the present embodiment is a system using the handpiece 1 of an ultrasonic treatment device in the first embodiment (see FIGS. 1 to 45) as a high-frequency treatment apparatus. The handpiece 1 in the present embodiment is connected to a high-frequency power supply main body 182. A P plate 183, which is a counter electrode plate used in a monopolar treatment apparatus, is connected to the high-frequency power supply main body 182. The P plate 183 is set above a bed used in treatment on which the patient P is placed. When the probe distal end 11a of the probe 11 of the handpiece 1 is used as a monopolar treatment apparatus, the handpiece 1 is used by sandwiching the patient P between the probe distal end 11a of the probe 11 and the P plate 183.

In a system according to the present embodiment, an output mode switching section 184, which is a switch configured to switch the output of the surgical apparatus between the bipolar mode and probe single output mode, is provided. The output mode switching section 184 switches the output of the surgical apparatus to the bipolar mode when tissues provided between the probe distal end 11a and the jaw 17 are griped by operating the movable handle 49 and the jaw 17 is practically closed with respect to the probe distal end 11a. In bipolar mode, a high-frequency current is passed to each of the probe distal end 11a and the jaw 17. In this state, each of the probe 11 and the jaw 17 in the handpiece 1 of the surgical apparatus is driven as a high-frequency electrode. Accordingly, tissues can be gripped between the probe distal end 11a and the jaw 17 in strong coagulation treatment.

When the jaw 17 is not practically closed with respect to the probe tip 11a (open state of the jaw 17), the output mode switching section 184 switches the output of the surgical apparatus to the probe single output mode. In a system according to the present embodiment, a high-frequency current only is output as energy from the probe distal end 11a of the probe 11 in the probe single output mode. In this state, the handpiece 1 is used as a monopolar treatment apparatus. Then, the handpiece 1 is used in a state that the patient P is sandwiched between the probe distal end 11a of the probe 11 and the P plate 183 in monopolar treatment. Accordingly, quick incisions can be made without sandwiching tissues by the probe distal end 11a.

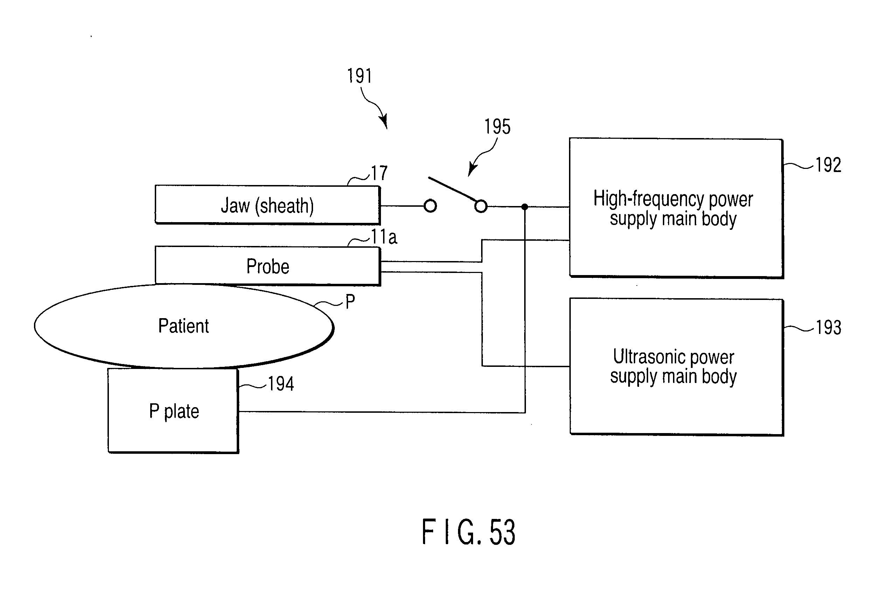

FIG. 53 is a diagram showing the fifth embodiment of the present invention. FIG. 53 is a block diagram schematically showing an electric circuit of a whole system of an ultrasonic treatment device according to the present embodiment. In the present embodiment, a surgical apparatus 191 constituted differently from the system of an ultrasonic treatment device in the first embodiment (see FIGS. 1 to 45) is provided. The configuration other than this changed portion (surgical apparatus 191) is substantially the same as that of the first embodiment (see FIGS. 1 to 45). Thus, in FIG. 53, the same reference numerals are attached to the same portions as those in the first embodiment and a description thereof is omitted.

The surgical apparatus 191 in the present embodiment is a system using the handpiece 1 of an ultrasonic treatment device in the first embodiment (see FIGS. 1 to 45) as a combined treatment apparatus combining high frequencies and ultrasonic waves. The handpiece 1 in the present embodiment is connected to a high-frequency power supply main body 192 and an ultrasonic power supply main body 193. A P plate 194, which is a counter electrode plate in a monopolar treatment apparatus, is connected to the high-frequency power supply main body 192. The P plate 194 is set above a bed used in treatment on which the patient P is placed. When the probe distal end 11a of the probe 11 of the handpiece 1 is used as a monopolar treatment apparatus, the handpiece 1 is used by sandwiching the patient P between the probe distal end 11a of the probe 11 and the P plate 194.

In a system according to the present embodiment, an output mode switching section 195, which is a switch configured to switch the output of the surgical apparatus between two modes (a first mode and a second mode), is provided. The output mode switching section 195 switches the output of the surgical apparatus to the first mode when the tissues between the probe distal end 11a and the jaw 17 are griped by operating the movable handle 49 and the jaw 17 is practically closed with respect to the probe distal end 11a.

The first mode is a mode in which a high-frequency treatment apparatus of the surgical apparatus is driven in the bipolar mode and at the same time, the ultrasonic vibrator 6 is driven. In the first mode, a high-frequency current is passed to the probe distal end 11a and the jaw 17. At the same time, ultrasonic vibrations are transmitted to the probe distal end 11a. In this state, each of the probe 11 and the jaw 17 in the handpiece 1 of the surgical apparatus is driven as a high-frequency electrode. Accordingly, tissues can be gripped between the probe distal end 11a and the jaw 17 in strong coagulation treatment and quick incisions.

When the jaw 17 is not practically closed with respect to the probe distal end 11a (open state of the jaw 17), the output mode switching section 195 switches the output to the second mode.

In the second mode, the output of the surgical apparatus is switched to the probe single output mode. In a system according to the present embodiment, ultrasonic vibrations are transmitted as energy from the probe distal end 11a of the probe 11 and also a high-frequency current is output in the probe single output mode. In this state, the handpiece 1 is used as a monopolar treatment apparatus. Then, the handpiece 1 is used in the state that the patient P is sandwiched between the probe distal end 11a of the probe 11 and the P plate 194 in monopolar treatment. In this case, ultrasonic vibrations are transmitted as energy from the probe distal end 11a of the probe 11 at the same time. Accordingly, quick incisions can be made without sandwiching tissues by the probe tip 11a. In this case, an incision without sticking of tissues to the probe distal end 11a and a puncture of tissues become possible.

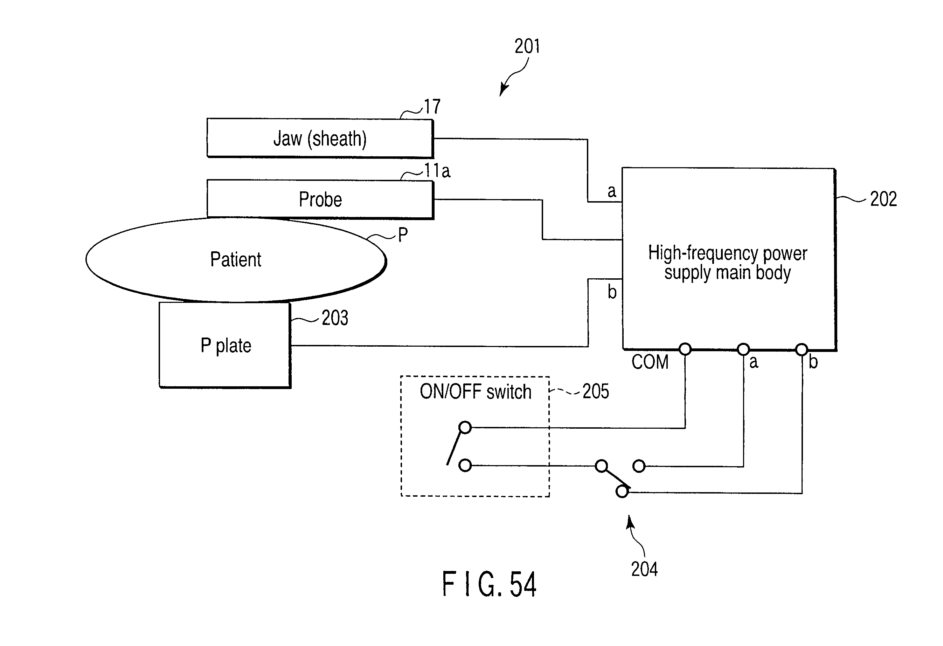

FIG. 54 is a diagram showing the sixth embodiment of the present invention. FIG. 54 is a block diagram schematically showing an electric circuit of a whole system of an ultrasonic treatment device according to the present embodiment. In the present embodiment, a surgical apparatus 201 constituted differently from the system of an ultrasonic treatment device in the first embodiment (see FIGS. 1 to 45) is provided. The configuration other than this changed portion (surgical apparatus 201) is substantially the same as that of the first embodiment (see FIGS. 1 to 45). Thus, in FIG. 54, the same reference numerals are attached to the same portions as those in the first embodiment and a description thereof is omitted.

The surgical apparatus 201 in the present embodiment is a system using the handpiece 1 of an ultrasonic treatment device in the first embodiment (see FIGS. 1 to 45) as a high-frequency treatment apparatus. The handpiece 1 in the present embodiment is connected to a high-frequency power supply main body 202. A P plate 203, which is a counter electrode plate in a monopolar treatment apparatus, is connected to the high-frequency power supply main body 202. When the probe distal end 11a of the probe 11 of the handpiece 1 is used as a monopolar treatment apparatus, the P plate 203 is set in a state that the patient P is sandwiched between the probe distal end 11a of the probe 11 and the P plate 203.

In a system according to the present embodiment, an output mode switching section 204, which is a switch configured to switch the output of the surgical apparatus between the bipolar mode and probe single output mode, is provided. Further, an on/off switch 205 is connected between the output mode switching section 204 and the high-frequency power supply main body 202.

The output mode switching section 204 switches the output of the surgical apparatus to the bipolar mode when the tissues between the probe distal end 11a and the jaw 17 are griped by operating the movable handle 49 and the jaw 17 is practically closed with respect to the probe distal end 11a. In bipolar mode, the electric circuit is switched to the terminal a side. Thus, a high-frequency current is passed to the probe distal end 11a and the jaw 17. In this state, each of the probe 11 and the jaw 17 in the handpiece 1 of the surgical apparatus is driven as a high-frequency electrode. Accordingly, tissues can be gripped between the probe distal end 11a and the jaw 17 in strong coagulation treatment. During the above operation, the conduction of a high-frequency current can be cut off by operating the on/off switch 205.

When the jaw 17 is not practically closed with respect to the probe distal end 11a (open state of the jaw 17), the output mode switching section 204 switches the output of the surgical apparatus to the probe single output mode. In a system according to the present embodiment, only a high-frequency current is output as energy from the probe distal end 11a of the probe 11 in the probe single output mode. In this state, the electric circuit is switched to the terminal b side. Thus, the handpiece 1 is used as a monopolar treatment apparatus. Then, the handpiece 1 is used in a state that the patient P is sandwiched between the probe distal end 11a of the probe 11 and the P plate 203 in monopolar treatment. Accordingly, quick incisions can be made without sandwiching tissues by the probe distal end 11a. During the above operation, the conduction of a high-frequency current can be cut off by operating the on/off switch 205.

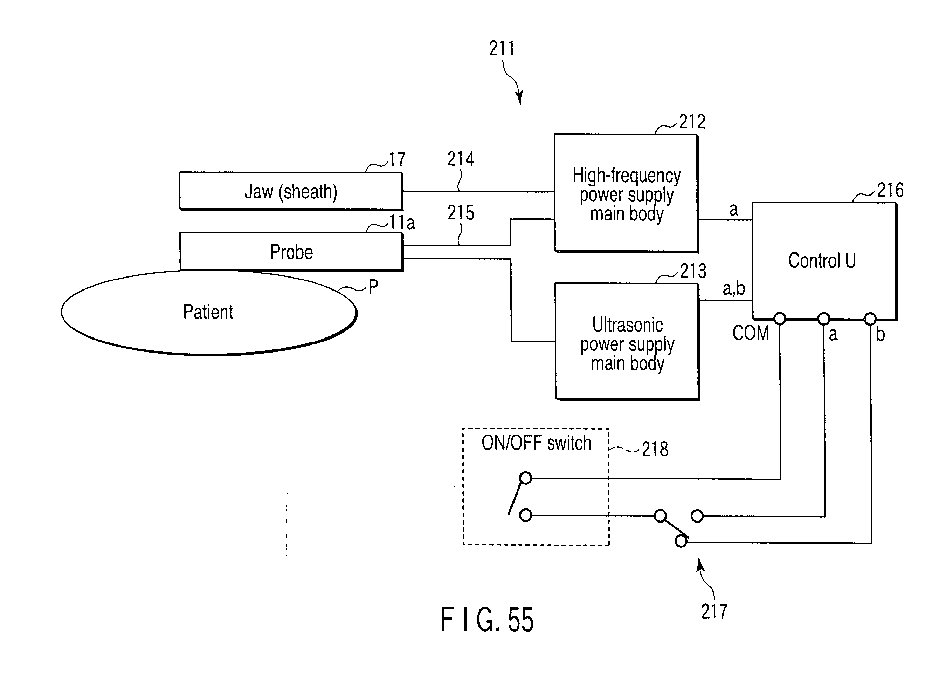

FIG. 55 is a diagram showing the seventh embodiment of the present invention. FIG. 55 is a block diagram schematically showing an electric circuit of a whole system of an ultrasonic treatment device according to the present embodiment. In the present embodiment, a surgical apparatus 211 constituted differently from the system of an ultrasonic treatment device in the first embodiment (see FIGS. 1 to 45) is provided. The configuration other than this changed portion (surgical apparatus 211) is substantially the same as that of the first embodiment (see FIGS. 1 to 45). Thus, in FIG. 55, the same reference numerals are attached to the same portions as those in the first embodiment and a description thereof is omitted.

The surgical apparatus 211 in the present embodiment is a system using the handpiece 1 of an ultrasonic treatment device in the first embodiment (see FIGS. 1 to 45) as a combined treatment apparatus combining high frequencies and ultrasonic waves. The handpiece 1 in the present embodiment is connected to a high-frequency power supply main body 212 and an ultrasonic power supply main body 213. A first electric path 214 connected to the jaw 17 and a second electric path 215 connected to the probe distal end 11a are connected to the high-frequency power supply main body 212. Further, the ultrasonic vibrator 6 of the handpiece 1 is connected to the ultrasonic power supply main body 213.

In a system according to the present embodiment, the high-frequency power supply main body 212 and the ultrasonic power supply main body 213 are connected to a control unit 216. An output mode switching section 217, which is a switch configured to switch the output of the surgical apparatus between two modes (a first mode and a second mode), is connected to the control unit 216. Further, an on/off switch 218 is connected between the output mode switching section 217 and the control unit 216.

The output mode switching section 217 switches the output of the surgical apparatus to the first mode when the tissues provided between the probe distal end 11a and the jaw 17 by operating the movable handle 49 and the jaw 17 is practically closed with respect to the probe distal end 11a.

The first mode is a mode in which a high-frequency treatment apparatus of the surgical apparatus is driven in the bipolar mode and at the same time, the ultrasonic vibrator 6 is driven. In the first mode, the electric circuit is switched to the terminal a side. Thus, a high-frequency current is passed to the probe distal end 11a and the jaw 17. At the same time, ultrasonic vibrations are transmitted to the probe distal end 11a. In this state, each of the probe 11 and the jaw 17 in the handpiece 1 of the surgical apparatus is driven as a high-frequency electrode. Accordingly, tissues can be gripped between the probe distal end 11a and the jaw 17 in strong coagulation treatment and quick incisions. During the above operation, the conduction can be cut off by operating the on/off switch 218.

When the jaw 17 is not practically closed with respect to the probe distal end 11a (open state of the jaw 17), the output mode switching section 217 switches the output to the second mode.

In the second mode, the output of the surgical apparatus is switched to the probe single output mode. In this state, the electric circuit is switched to the terminal b side. Thus, in a system according to the present embodiment, only ultrasonic vibrations are transmitted as energy from the probe distal end 11a of the probe 11 in the probe single output mode. Accordingly, for example, treatment such as a puncture is possible by using cavitation of the probe 11. During the above operation, the conduction can be cut off by operating the on/off switch 218.

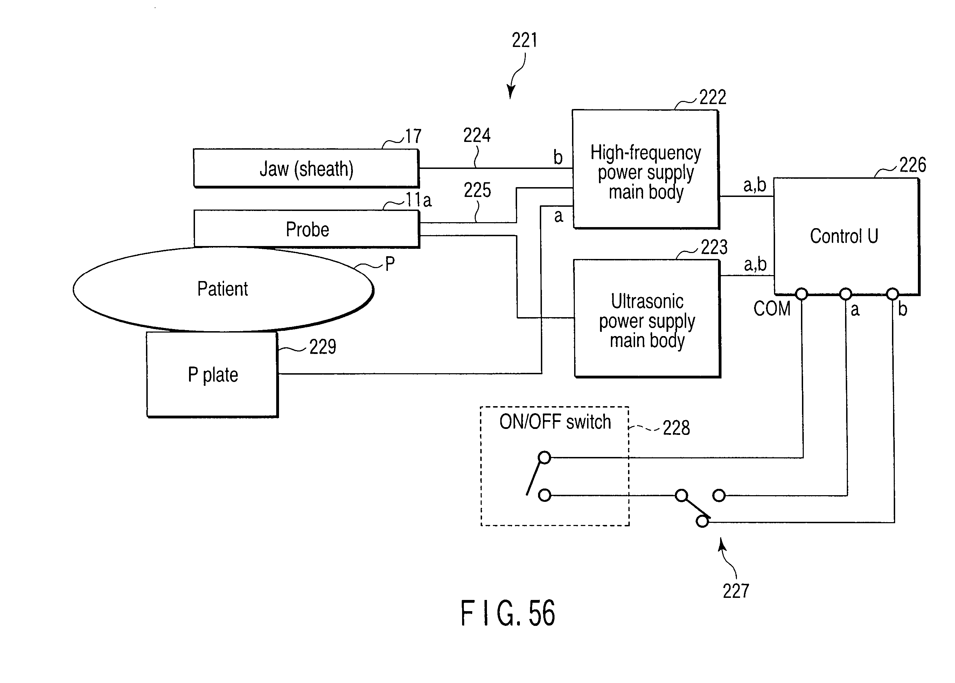

FIG. 56 is a diagram showing the eighth embodiment of the present invention. FIG. 56 is a block diagram schematically showing an electric circuit of a whole system of an ultrasonic treatment apparatus according to the present embodiment. In the present embodiment, a surgical apparatus 221 constituted differently from the system of an ultrasonic treatment device in the first embodiment (see FIGS. 1 to 45) is provided. The configuration other than this changed portion (surgical apparatus 221) is substantially the same as that of the first embodiment (see FIGS. 1 to 45). Thus, in FIG. 56, the same reference numerals are attached to the same portions as those in the first embodiment and a description thereof is omitted.

The surgical apparatus 221 in the present embodiment is a system using the handpiece 1 of an ultrasonic treatment device in the first embodiment (see FIGS. 1 to 45) as a combined treatment apparatus combining high frequencies and ultrasonic waves. The handpiece 1 in the present embodiment is connected to a high-frequency power supply main body 222 and an ultrasonic power supply main body 223. A first electric path 224 connected to the jaw 17, a second electric path 225 connected to the probe distal end 11a, and a P plate 229, which is a counter electrode plate in a monopolar treatment apparatus, are connected to the high-frequency power supply main body 222. Further, the ultrasonic vibrator 6 of the handpiece 1 is connected to the ultrasonic power supply main body 223.

In a system according to the present embodiment, the high-frequency power supply main body 222 and the ultrasonic power supply main body 223 are connected to a control unit 226. An output mode switching section 227, which is a switch configured to switch the output of the surgical apparatus between two modes (a first mode and a second mode), is connected to the control unit 226. Further, an on/off switch 228 is connected between the output mode switching section 227 and the control unit 226.

The output mode switching section 227 switches the output of the surgical apparatus to the first mode when the tissues between the probe distal end 11a and the jaw 17 by operating the movable handle 49 and the jaw 17 is practically closed with respect to the probe distal 11a.

The first mode is a mode in which a high-frequency treatment apparatus of the surgical apparatus is driven in the bipolar mode and at the same time, the ultrasonic vibrator 6 is driven. In the first mode, the electric circuit is switched to the terminal a side. Thus, a high-frequency current is passed to the probe distal end 11a and the jaw 17. At the same time, ultrasonic vibrations are transmitted to the probe distal end 11a. In this state, each of the probe 11 and the jaw 17 in the handpiece 1 of the surgical apparatus is driven as a high-frequency electrode. Accordingly, tissues can be gripped between the probe tip 11a and the jaw 17 in strong coagulation treatment and quick incisions. During the above operation, the conduction can be cut off by operating the on/off switch 228.

When the jaw 17 is not practically closed with respect to the probe distal end 11a (open state of the jaw 17), the output mode switching section 227 switches the output to the second mode.

In the second mode, the output of the surgical apparatus is switched to the probe single output mode. In this state, the electric circuit is switched to the terminal b side. Thus, in a system according to the present embodiment, ultrasonic vibrations are transmitted as energy from the probe distal end 11a of the probe 11 and also a high-frequency current is output in the probe single output mode. In this case, the handpiece 1 is used as a monopolar treatment apparatus. Then, the handpiece 1 is used in a state that the patient P is sandwiched between the probe distal end 11a of the probe 11 and the P plate 229 in monopolar treatment. In this case, ultrasonic vibrations are transmitted as energy from the probe distal end 11a of the probe 11 at the same time. Accordingly, quick incisions can be made without sandwiching tissues by the probe distal end 11a. Also, an incision without sticking of tissues to the probe distal end 11a and a puncture of tissues become possible. During the above operation, the conduction can be cut off by operating the on/off switch 228.

Incidentally, the present invention is not limited to the above embodiments and various modifications can naturally be made without deviating from the spirit and scope of the present invention.

## Claims

1. A surgical apparatus (1, 181, 191, 201, 211, 221) comprising:
a probe (11) configured to output energy of treating an organism;
a jaw (17) which can be opened/closed with respect to the probe (11); and
an output mode switching section (141, 151, 161, 184, 195, 204, 217, 227) configured to selectively switch an output mode between a bipolar mode in which the probe (11) and the jaw (17) are driven as high-frequency electrodes and a probe single output mode in which the energy is output from only the probe (11) in accordance with an open/closed state of the jaw (17).

2. The surgical apparatus (1, 181, 191, 201, 211, 221) according to claim 1, further comprising:
an operation section (47, 49) configured to operate the jaw (17) to open/close,
wherein the output mode switching section (141, 151, 161, 184, 195, 204, 217, 227) includes an output mode switching section (141, 151, 161, 184, 195, 204, 217, 227) which is configured to switch the output mode to the bipolar mode when tissues are griped between the probe (11) and the jaw (17) by an operation of the operation section (47, 49) and the jaw (17) is closed with respect to the probe (11), and which is configured to switch the output mode to the probe single output mode when the jaw (17) is in an open state in which the jaw (17) is not closed with respect to the probe (11).

3. The surgical apparatus (181, 201) according to claim 1, wherein the output mode switching section (141, 151, 161, 184, 195, 204, 217, 227) includes an output mode switching section (184, 204) configured to cause the probe (11) to output only a high-frequency current as the energy in the probe single output mode.

4. The surgical apparatus (1, 191, 211, 221) according to claim 1, further comprising: an ultrasonic output section (2, 8a, 193, 213, 223) configured to supply ultrasonic waves to the probe (11).

5. The surgical apparatus (1, 211) according to claim 4, wherein the output mode switching section (141, 151, 161, 195, 217, 227) includes an output mode switching section (141, 151, 161, 217) configured to cause the probe (11) to output only the ultrasonic waves from the ultrasonic output section (2, 8a, 213) as the energy in the probe single output mode.

6. The surgical apparatus (191, 221) according to claim 4, wherein the output mode switching section (141, 151, 161, 195, 217, 227) includes an output mode switching section (195, 227) configured to cause the probe (11) to output a high-frequency current and the ultrasonic waves at the same time as the energy in the probe single output mode.

7. The surgical apparatus (1, 191, 211, 221) according to claim 4, wherein the output mode switching section (141, 151, 161, 195, 217, 227) includes an output mode switching section (141, 151, 161, 195, 217, 227) configured to cause the probe (11) to further output the ultrasonic waves at the same time as the energy in the bipolar mode.

8. A surgical apparatus (1, 181, 191, 201, 211, 221) comprising:
a probe (11) configured to output energy of treating an organism;
a jaw (17) which can be opened/closed with respect to the probe (11);
an operation section (47, 49) configured to operate the jaw (17) to open/close; and
an output mode switching section (141, 151, 161, 184, 195, 204, 217, 227) configured to selectively switch an output mode to one of a probe single output mode in which the energy is output only from the probe (11) and a bipolar mode in which each of the probe (11) and the jaw (17) is driven as a high-frequency electrode,
wherein the output mode switching section (141, 151, 161, 184, 195, 204, 217, 227) includes:
a movable member (34, 64, 65, 67, 98, 134a, 134b, 152, 164, 165, 175) configured to move in accordance with an operation of the operation section (47, 49); and
a contact switching section (142, 153, 171) configured to perform an opening/closing operation of a contact, in which a conduction state of a high-frequency current to the jaw (17) is connected or cut off, in accordance with a movement of the movable member (34, 64, 65, 67, 98, 134a, 134b, 152, 164, 165, 175).

9. The surgical apparatus (1, 181, 191, 201, 211, 221) according to claim 8, wherein the contact switching section (142, 153, 171) includes a contact switching section (142, 153, 171) which is configured to perform the closing operation of the contact to switch to a state in which conduction of the high-frequency current to the jaw (17) is established in accordance with the movement of the movable member (34, 64, 65, 67, 98, 134a, 134b, 152, 164, 165, 175) when the operation to move the jaw (17) in a closing direction is performed by the operation section (47, 49), and which is configured to perform the opening operation of the contact to switch to the state in which the conduction of the high-frequency current to the jaw (17) is cut off in accordance with the movement of the movable member (34, 64, 65, 67, 98, 134a, 134b, 152, 164, 165, 175) when the operation to move the jaw (17) in an opening direction is performed by the operation section (47, 49).

10. The surgical apparatus (1, 181, 191, 201, 211, 221) according to claim 8, wherein
the movable member (34, 64, 65, 67, 98, 134a, 134b, 152, 164, 165, 175) includes an operation force transmission member (34, 64, 65, 67, 98) configured to be driven to advance or retreat in an axial direction of the probe (11) in accordance with the operation of the operation section (47, 49) and configured to transmit an operation force of the operation section (47, 49) to the jaw (17), and
the contact switching section (142, 153, 171) includes a contact switching section (142, 153) configured to perform the opening/closing operation of the contact in accordance with an advancing/retreating movement of the operation force transmission member (34, 64, 65, 67, 98).

11. The surgical apparatus (1, 181, 191, 201, 211, 221) according to claim 10, wherein the contact switching section (142, 153) includes a contact switching section (142, 153) which is configured to switch conduction of the high-frequency current to the jaw (17) to a connected state by performing the closing operation of the contact in accordance with the advancing/retreating movement of the operation force transmission member (34, 64, 65, 67, 98) when the jaw (17) is moved in a closing direction by the operation section (47, 49), and which is configured to switch the conduction of the high-frequency current to the jaw (17) to a state of cutoff by performing the opening operation of the contact in accordance with the advancing/retreating movement of the operation force transmission member (34, 64, 65, 67, 98), when the jaw (17) is moved in an opening direction by the operation section (47, 49).

12. The surgical apparatus (1, 181, 191, 201, 211, 221) according to claim 8, wherein
the operation section (47, 49) includes a fixed handle (47) and a movable handle (49) configured to be movable in a direction toward or away from the fixed handle (47),
the movable member (34, 64, 65, 67, 98, 134a, 134b, 152, 164, 165, 175) includes a movable member (164, 165, 175) provided in the operation section (47, 49), and
the contact switching section (142, 153, 171) includes a contact switching section (171) which is configured to switch conduction of the high-frequency current to the jaw (17) to a connected state by performing the closing operation of the contact in accordance with a movement of the movable handle (49) when the jaw (17) is operated to move in a closing direction, and which is configured to switch the conduction of the high-frequency current to the jaw (17) to a state of cutoff by performing the opening operation of the contact in accordance with the movement of the movable handle (49) when the jaw (17) is operated to move in an opening direction.

13. The surgical apparatus (181, 201) according to claim 8, wherein the output mode switching section (141, 151, 161, 184, 195, 204, 217, 227) includes an output mode switching section (184, 204) configured to cause the probe (11) to output only the high-frequency current as the energy in the probe single output mode.

14. The surgical apparatus (1, 191, 211, 221) according to claim 8, further comprising: an ultrasonic output section (2, 8a, 193, 213, 223) configured to supply ultrasonic waves to the probe (11).

15. The surgical apparatus (1, 211) according to claim 14, wherein the output mode switching section (141, 151, 161, 195, 217, 227) includes an output mode switching section (141, 151, 161, 217) configured to cause the probe (11) to output only the ultrasonic waves from the ultrasonic output section (2, 8a, 213) as the energy in the probe single output mode.

16. The surgical apparatus (191, 221) according to claim 14, wherein the output mode switching section (141, 151, 161, 195, 217, 227) includes an output mode switching section (195, 227) configured to cause the probe (11) to output high frequencies and the ultrasonic waves at the same time as the energy in the probe single output mode.

17. The surgical apparatus (1, 191, 211, 221) according to claim 14, wherein the output mode switching section (141, 151, 161, 195, 217, 227) includes an output mode switching section (141, 151, 161, 195, 217, 227) configured to cause the probe (11) to further output the ultrasonic waves at the same time as the energy in the bipolar mode.

18. A surgical apparatus (1, 181, 191, 201, 211, 221) comprising:
a probe (11) configured to output energy of treating an organism;
a jaw (17) which can be opened/closed with respect to the probe (11); and
an output mode switching mechanism (141, 151, 161, 184, 195, 204, 217, 227) configured to switch an output mode between a bipolar mode in which the probe (11) and the jaw (17) are used as electrodes and a probe single output mode in which the energy is output from only the probe (11),
wherein the output mode switching mechanism (141, 151, 161, 184, 195, 204, 217, 227) includes an output mode switching mechanism (141, 151, 161, 184, 195, 204, 217, 227) configured to switch to the bipolar mode when the jaw (17) performs a closing operation and configured to switch to the probe single output mode when the jaw (17) performs an opening operation in accordance with an opening/closing operation of the jaw (17).
